# EUROPEAN PATENT SPECIFICATION

(11) **EP 4 208 230 B1**
(45) Date of publication and mention of the grant of the patent: **25.03.2026**
(21) Application number: 21773478.9
(22) Date of filing: 01.09.2021
(51) Int. Cl.: A61M 15/08, A61M 11/00, A61M 15/00, B05B 11/02, B05B 12/08, G16H 20/13, G16H 40/63, A61J 1/06, A61J 1/14, A61J 1/16, A61M 16/16, H04W 12/47

(54) **DRUG DELIVERY DEVICES WITH ON-BOARD DRUG DESTRUCTION**
ARZNEIMITTELABGABEVORRICHTUNGEN MIT INTEGRIERTER ARZNEIMITTELZERSTÖRUNG
DISPOSITIFS D'ADMINISTRATION DE MÉDICAMENT AVEC DESTRUCTION DE MÉDICAMENT INTÉGRÉE

(30) Priority: 01.09.2020 US 202063073016 P
(43) Date of publication of application: 12.07.2023
(73) Proprietor: Janssen Pharmaceutica NV, 2340 Beerse (BE)
(72) Inventor: HUBERT, Emma Louise, Milpitas, California 95035 (US); KAPIL, Monica A., San Jose, California 95127 (US); ZHAO, MingQi, Milpitas, California 95035 (US); VESOLE, Steven M., Milpitas, California 95035 (US); SCRIMGEOUR, Ian, Dunbar EH42 ILT (GB); RAMOS, David, Orange Park, Florida 32065 (US); SINGH, Jaskaran, San Diego, California 92127 (US); WANG, Jingli, Milpitas, California 95035 (US); KALIKHMAN, David, Titusville, New Jersey 08560 (US); POPLI, Shagun, Milpitas, California 95035 (US); KRULEVITCH, Peter, San Antonio, Texas 78212 (US); YAN, Hong, Milpitas, California 95035 (US); BARATTA, Michael A., Horsham, Pennsylvania 19382 (US); PHILLIPS, Whitney, Camas, Washington 98607 (US); ALBERTINI, Francesco N., 8200 Schaffhausen (CH); CANNAMELA, Michael, New York, New York 10001 (US)
(74) Representative: Carpmaels & Ransford LLP
(86) International application number: PCT/EP2021/074091
(87) International publication number: WO 2022/049102

(56) References cited:
- WO-A1-2015/025324
- WO-A2-2007/081947
- US-A1- 2018 110 725
- US-A1- 2020 197 633
- US-B1- 10 737 041
- US-B2- 9 061 879

## Description

### FIELD

The present disclosure relates generally to drug delivery devices with on-board drug destruction and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration. After the drug is delivered, the device may be disposed of as a used device for recycling or as medical waste. However, the device can have a residual amount of the drug remaining in the device that cannot be further delivered from the device. The residual drug may not be intended by the device manufacturer and/or the drug prescriber to be accessed by a person, but the residual drug may nevertheless be accessed, e.g., by accident or by breaking the device to access the residual drug. Thus, the residual drug may be accessed by an unauthorized person, such as a child, a person not prescribed the drug, etc., who could be harmed by the drug and/or attempt its unauthorized use. Preventing access to the residual drug may be particularly important for controlled substances. Similarly, if device is diverted from its intended destination during the shipping process, either by accident or by intention, the drug in the device may be accessed by an unauthorized person. WO2007/081947A2 describes drug storage and dispensing devices and systems. US10737041B1 describes secure electronic vaporizer and nebulizer systems. US9061879B2 describes a secure liquid drug dispenser and method for delivering liquid medication. US2018/110725A1 describes compositions, methods and kits for the safe inhaled delivery of targeted opioids for the treatment of pain and addiction. WO2015/025324A1 describes a device to deliver a predetermined amount of a substance to a natural orifice of the body. US2020/197633A1 describes a blow-fill-seal devices and methods for delivering a substance to a body cavity.

Accordingly, there remains a need for improved nasal drug delivery devices.

### SUMMARY

The invention is defined by the appended claims. In the following, embodiments of the invention and embodiments useful to understand the invention are described.

In general, drug delivery devices with on-board drug destruction, drug products utilizing the same, and methods of using drug delivery devices with on-board drug destruction are provided.

In one aspect, a drug delivery system is provided that in one embodiment includes a tip configured to be positioned in a nose of a patient. The tip has an opening therein. The drug delivery system also includes a drug holder containing a drug therein that is configured to exit through the opening, and a destruction mechanism configured to be activated and thereby destroy substantially all of the drug in the drug holder.

The drug delivery system can have any number of variations. For example, the destruction mechanism can include a chemical. The chemical can include one of silica, bleach, charcoal, glycerol, sodium polyacrylate, activated carbon, zeolytes, and acid. The drug delivery system can also include a barrier configured to prevent the drug and the chemical from coming into contact with one another, and the activation of the destruction mechanism causes the barrier to break to allow the drug and the chemical to come into contact with one another.

For another example, the destruction mechanism can include an absorbable material configured to absorb the drug therein. The absorbable material can include silica or a sponge.

For yet another example, the destruction mechanism can be disposed in the drug holder. For still another example, the drug delivery system can also include a body with the drug holder disposed therein, and the destruction mechanism can be disposed in the body external to the drug holder. For another example, the destruction mechanism can be attached to a package containing the drug holder therein.

For yet another example, the drug delivery system can also include an actuator configured to be actuated by a user to cause the drug to be delivered out of the opening. After the activation of the destruction mechanism, the actuation of the actuator can be configured to not cause the drug to be delivered out of the opening.

For another example, the drug delivery system can also include a processor configured to cause the activation of the destruction mechanism in response to occurrence of a predetermined trigger event. The drug delivery system can also include a body with the drug holder disposed therein, and the processor can be disposed in the body. The drug delivery system can also include a communications interface configured to electronically receive an instruction from an external device, and the external device can include the processor. The drug delivery system can also include a location sensor configured to monitor geographic location, a temperature sensor configured to monitor temperature, a humidity sensor configured to monitor humidity, a pH sensor configured to monitor pH, and/or an ultraviolet (UV) sensor configured to monitor UV exposure. The predetermined trigger event can include the geographic location sensed by the location sensor being an unacceptable geographic location as determined by the processor. The predetermined trigger event can include the temperature sensed by the temperature sensor being an unacceptable temperature as determined by the processor. The predetermined trigger event can include the humidity sensed by the humidity sensor being an unacceptable humidity as determined by the processor. The predetermined trigger event can include the pH sensed by the pH sensor being an unacceptable pH as determined by the processor. The predetermined trigger event can include the UV exposure sensed by the UV sensor being an unacceptable UV exposure as determined by the processor. The predetermined trigger event can include the processor identifying an unacceptable Drug Enforcement Administration (DEA) Registration Number. The drug delivery system can also include a tag storing data therein, and the predetermined trigger event can include the processor identifying unacceptable data received from the tag.

For still another example, the destruction mechanism can be configured to be automatically activated in response to occurrence of a predetermined trigger event. The drug delivery system can also include a location sensor configured to monitor geographic location, a temperature sensor configured to monitor temperature, a humidity sensor configured to monitor humidity, a pH sensor configured to monitor pH, and/or an ultraviolet (UV) sensor configured to monitor UV exposure. The predetermined trigger event can include the geographic location sensed by the location sensor being an unacceptable geographic location. The predetermined trigger event can include the temperature sensed by the temperature sensor being an unacceptable temperature. The predetermined trigger event can include the humidity sensed by the humidity sensor being an unacceptable humidity. The predetermined trigger event can include the pH sensed by the pH sensor being an unacceptable pH. The predetermined trigger event can include the UV exposure sensed by the UV sensor being an unacceptable UV exposure. The predetermined trigger event can include identification of an unacceptable Drug Enforcement Administration (DEA) Registration Number. The drug delivery system can also include a tag storing data therein, and the predetermined trigger event can include identification of unacceptable data received from the tag.

For yet another example, the drug can be one of ketamine, esketamine, naloxone, and sumatriptan.

In another aspect, a drug product is provided that in one embodiment includes a drug product disposed in a drug delivery device. The drug delivery device includes a tip configured to be positioned in a nose of a patient. The tip has an opening therein. The drug delivery system also includes a drug holder containing the drug product therein that is configured to exit through the opening, and a destruction mechanism configured to be activated and thereby destroy substantially all of the drug product in the drug holder. The drug product is one of ketamine, esketamine, naloxone, and sumatriptan. The drug delivery device can have any number of variations.

In another aspect, a drug delivery method (not claimed) is provided that in one embodiment includes activating a destruction mechanism on board a nasal drug delivery device and thereby destroying substantially all drug contained in a drug holder on board the drug delivery device.

The drug delivery method can have any number of variations. For example, the destruction mechanism can include a chemical, and the activation of the destruction mechanism can cause the chemical and the drug to come into contact with one another. The chemical can include one of silica, bleach, charcoal, glycerol, sodium polyacrylate, activated carbon, zeolytes, and acid.

For another example, the destruction mechanism can includes an absorbable material, and the activation of the destruction mechanism causes the absorbable material to absorb the drug therein. The absorbable material can include silica or a sponge.

For yet another example, the destruction mechanism can be disposed in the drug holder. For still another example, the destruction mechanism can be located on board the drug delivery device outside of the drug holder. For another example, the destruction mechanism can be disposed external to and separate from the drug delivery device. For still another example, the drug delivery method can also include preventing the drug from being delivered out of an opening of the drug delivery device after the activation of the destruction mechanism.

For yet another example, a processor can cause the activation of the destruction mechanism in response to occurrence of a predetermined trigger event. The processor can be on board the drug delivery device. The processor can be external to and separate from the drug delivery device. The drug delivery method can also include sensing a geographic location using a location sensor, sensing a temperature using a temperature sensor, sensing a humidity using a humidity sensor, sensing a pH using a pH sensor, and/or sensing ultraviolet (UV) exposure using a UV sensor. The predetermined trigger event can include the processor determining that the sensed geographic location is an unacceptable geographic location. The predetermined trigger event can include the processor determining that the sensed temperature is an unacceptable temperature. The predetermined trigger event can include the processor determining that the sensed humidity is an unacceptable humidity. The predetermined trigger event can include the processor determining that the sensed pH is an unacceptable pH. The predetermined trigger event can include the processor determining that the sensed UV exposure is an unacceptable UV exposure. The predetermined trigger event can include the processor identifying an unacceptable Drug Enforcement Administration (DEA) Registration Number. The drug delivery method can also include causing the processor to receive data read from a tag, and the predetermined trigger event can include the processor identifying unacceptable data read from the tag.

For another example, the destruction mechanism can be automatically activated in response to occurrence of a predetermined trigger event. For yet another example, the drug can be one of ketamine, esketamine, naloxone, and sumatriptan.

### BRIEF DESCRIPTION OF DRAWINGS

The present disclosure is described by way of reference to the accompanying figures which are as follows:
FIG. 1 is a block diagram of one embodiment of a drug delivery device;
FIG. 2 is a side cross-sectional view of one embodiment of a vial;
FIG. 3 is a side cross-sectional view of another embodiment of a vial of an embodiment of the invention;
FIG. 4 is a side view of one embodiment of a barrier of the vial of FIG. 3 in an unbroken state;
FIG. 5 is a side view of the barrier of FIG. 5 in a broken state;
FIG. 6 is a side cross-sectional view of yet another embodiment of a vial;
FIG. 7 is a top view of the vial of FIG. 6;
FIG. 8 is a side cross-sectional view of a portion of one embodiment, according to the invention, of the drug delivery device of FIG. 1;
FIG. 9 is a side cross-sectional view of the portion of the drug delivery device of FIG. 8 after drug delivery;
FIG. 10 is a side cross-sectional view of the portion of the drug delivery device of FIG. 8 before drug delivery with a barrier in a broken state;
FIG. 11 is a side cross-sectional view of a portion of another embodiment, according to the invention, of the drug delivery device of FIG. 1;
FIG. 12 is a side cross-sectional view of the portion of the drug delivery device of FIG. 11 before drug delivery with a barrier in a broken state;
FIG. 13 is a side cross-sectional view of one embodiment of a housing with a cover and containing a substance therein;
FIG. 14 is a perspective view of the housing and cover of FIG. 13;
FIG. 15 is a side cross-sectional view of the housing and cover of FIG. 13 with the substance escaping through holes of the housing and cover;
FIG. 16 is a perspective view of one embodiment of a box;
FIG. 17 is a side cross-sectional view of a portion of yet another embodiment of the drug delivery device of FIG. 1;
FIG. 18 is a side cross-sectional view of the portion of the drug delivery device of FIG. 17 with a melted substance;
FIG. 19 is a block diagram of another embodiment of a drug delivery device;
FIG. 20 is a side cross-sectional view of a portion of one embodiment, according to the invention, of the drug delivery device of FIG. 19;
FIG. 21 is a side cross-sectional view of the portion of the drug delivery device of FIG. 20 with an escaping substance;
FIG. 22 is a side cross-sectional view of a portion of another embodiment, according to the invention, of the drug delivery device of FIG. 19;
FIG. 23 is a side cross-sectional view of the portion of the drug delivery device of FIG. 22 with an inflated bag;
FIG. 24 is a side view of one embodiment of the drug delivery device of FIG. 1 or FIG. 17;
FIG. 25 is a side view of another embodiment of the drug delivery device of FIG. 1 or FIG. 19, the drug delivery device being in a pre use state;
FIG. 26 is a side view of the drug delivery device of FIG. 25 in a post use state;
FIG. 27 is a side cross-sectional view of yet another embodiment of the drug delivery device of FIG. 1 or FIG. 19; and
FIG. 28 is a schematic view of one embodiment of a computer system.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention if they fall within the scope of the claims.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with on-board drug destruction, drug products utilizing the same, and methods of using drug delivery devices with on-board drug destruction are provided. In general, a nasal drug delivery device configured to dispense a drug therefrom into a nose includes a destruction mechanism configured to destroy drug contained in the drug delivery device. In some embodiments, the destruction mechanism is configured to destroy the drug by affecting the drug chemically, e.g., by introducing the drug to a chemical such as acid, bleach, a diluting liquid, etc., such that the drug is no longer potent and/or stable such that the drug is destroyed by losing its effectiveness as a treatment. After such drug destruction, even if the drug delivery device is actuated and delivers a spray therefrom, the sprayed droplets will not be effective as a drug as intended. In some embodiments, the destruction mechanism is configured to destroy the drug by absorbing the drug as a liquid into silica, a sponge, or other absorbable material such that the drug is destroyed by no longer being deliverable from the drug delivery device as a nasal spray. Regardless of the configuration of the destruction mechanism, the drug delivery device can be configured to prevent actuation thereof after the destruction mechanism destroys the drug, which may further help prevent access to the drug and/or may prevent an unsafe chemical from being sprayed out of the drug delivery device.

The destruction mechanism can be configured to destroy the drug before a first actuation of the drug delivery device to deliver the drug therefrom such that the destruction mechanism destroys substantially all of the drug contained in the drug delivery device. The drug can therefore be prevented from being accessed by an unauthorized person, such as a child, a person not prescribed the drug, etc., who could be harmed by the drug and/or attempt its unauthorized use. Preventing access to the drug may be particularly important for esketamine, ketamine, and other controlled substances. The drug may be desirable to destroy before any delivery thereof from the drug delivery device to help ensure that the drug is not accessed by an unauthorized person, e.g., during the shipping process before the drug delivery device reaches its intended destination, and/or to help ensure that the drug is delivered before the drug's expiration and while the drug is potent and stable.

Alternatively or in addition, the destruction mechanism can be configured to destroy the drug after a first actuation of the drug delivery device to deliver the drug therefrom such that the destruction mechanism destroys substantially all of the drug remaining in the drug delivery device. The destruction of the drug after the first actuation can be in instances where the drug delivery device is intended to be actuated only once to deliver drug from the drug delivery device such that the destruction mechanism destroys substantially all residual drug remaining in the drug delivery device. The residual drug can therefore be prevented from being accessed by an unauthorized person, such as a child, a person not prescribed the drug, etc., who could be harmed by the drug and/or attempt its unauthorized use. Preventing access to the residual drug may be particularly important for esketamine, ketamine, and other controlled substances. Some drugs, such as esketamine, ketamine, and other controlled substances, may be required to be disposed of per a government requirement, e.g., U.S. federal Drug Enforcement Administration requirement, non-U.S. federal requirement, state requirement, or local requirement, to help, e.g., ensure that the drug is not accessed by an unauthorized person. Having substantially no residual drug left in a drug delivery device after use thereof may facilitate recycling of the used drug delivery device since substantially no drug will be present when the drug delivery device is recycled. In other instances, the destruction of the drug after the first actuation can be in instances where the drug delivery device is intended to be actuated more than once to deliver drug from the drug delivery device such that the destruction mechanism either destroys substantially all residual drug remaining in the drug delivery device after all of the intended actuations or destroys substantially all drug remaining in the drug delivery device despite one or more intended drug deliveries being available from the drug delivery device. Destroying the drug despite one or more possible drug deliveries being possible from the drug delivery device may help ensure that the drug is not accessed by an unauthorized person and/or that the drug is delivered before the drug's expiration and while the drug remains potent and stable.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient can have a variety of configurations. In general, the drug delivery device is configured to deliver a drug to a patient, where the drug is provided in a defined dosage form within the drug delivery device.

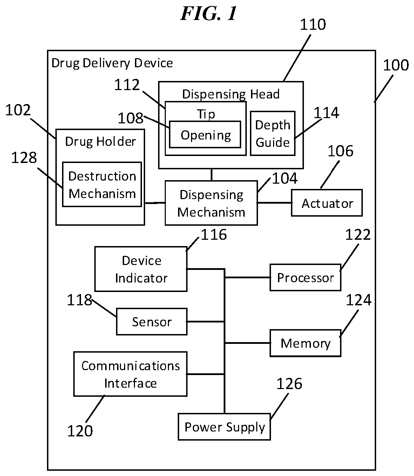

FIG. 1 illustrates one embodiment of a drug delivery device 100 configured to expel a drug into a nose of a patient and configured to destroy drug on board the drug delivery device 100 using a destruction mechanism 128. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, such as a cartridge, a vial, a blow-fill-seal (BFS) capsule, a blister pack, etc. In an exemplary embodiment, the drug holder 102 is a vial. An exemplary vial is formed of one or more materials, e.g., glass, polymer(s), etc. In some embodiments, a vial can be formed of glass. In other embodiments, a vial can be formed of one or more polymers. In yet other embodiments, different portions of a vial can be formed of different materials.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston, pin, and/or a needle configured to pierce through or puncture a seal member of the drug holder 102 in embodiments in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a pressable button. For another example, the actuator 106 can include a movable switch. For yet another example, the actuator 106 can include a squeezable body of the drug holder 102.

The destruction mechanism 128 is configured to destroy the drug contained in the drug holder 102. The drug holder 102 includes the destruction mechanism 128 in this illustrated embodiment. The destruction mechanism 128 can have a variety of configurations, as discussed further below.

The destruction mechanism 128 can be configured to destroy the drug contained in the drug holder 102 after actuation of the actuator 106. In this way, the destruction mechanism 128 can be configured to destroy substantially all residual drug left in the drug holder 102 after completion of drug delivery. A person skilled in the art will appreciate that the residual drug may not be 100% destroyed but nevertheless be considered to be substantially all destroyed due to any number of factors, such as sensitivity of measurement equipment. It may be beneficial to have zero residual drug left in a drug delivery device after use thereof to help ensure that any residual drug is not accessed by any unauthorized persons, which may be particularly important when the drug is prescribed to a particular patient only for use by the patient and/or when the drug is a controlled substance. Some drugs, such as controlled substances, may be required to be disposed of per a government requirement to help, e.g., ensure that the drug is not accessed by an unauthorized person. Having substantially no residual drug left in a drug delivery device after use thereof may facilitate recycling of the used drug delivery device since no drug will be present when the drug delivery device is recycled.

Alternatively or in addition, the destruction mechanism 128 can be configured to destroy the drug contained in the drug holder 102 before actuation of the actuator 106. In this way, the destruction mechanism 128 can be configured to destroy substantially all drug in the drug holder 102 before drug delivery begins. A person skilled in the art will appreciate that the drug may not be 100% destroyed but nevertheless be considered to be substantially all destroyed due to any number of factors, such as sensitivity of measurement equipment. If the drug holder 102 is diverted during its shipping process from an expected geographic location and/or an expected vendor (e.g., a warehouse, a pharmacy, a hospital, a medical clinic, etc.) in the supply chain, destroying the drug in the drug holder 102 before actuation of the actuator 106 may help ensure that the drug in the drug holder 102 is not accessed by any unauthorized persons, which may be particularly important when the drug is prescribed to a particular patient only for use by the patient, when the drug is a controlled substance, and/or when proof of supply chain compliance is required for a particular drug as part of the particular drug's Risk Evaluation and Mitigation Strategies (REMS), e.g., a REMS for a controlled substance. If the actuator 106 is not actuated within a predetermined amount of time, e.g., a predetermined amount of time from a patient's receipt of the drug holder 102, a predetermined amount of time from shipping of the drug holder 102 from a manufacturer of the drug holder 102, a predetermined amount of time from receipt of the drug holder 102 by a hospital or other health care facility, etc., destroying the drug in the drug holder 102 before actuation of the actuator 106 may help ensure that the drug in the drug holder 102 is not accessed by any unauthorized persons and/or that the drug is delivered before its expiration and during its effective life, e.g., that the drug is used during its effective life as defined by the predetermined amount of time.

The opening 108 through which the drug exits the drug delivery device 100 is formed in a dispensing head 110 of the drug delivery device 100 in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

In some embodiments, the dispensing head 110 can include two tips 112 each having an opening 108 therein such that the drug delivery device 100 is configured to simultaneously deliver doses of drug into two nostrils in response to a single actuation of the actuator 106.

In some embodiments, the actuator 106 is configured to be actuated only once to deliver a dose of the drug to a patient.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100, the drug contained in the drug holder 102, and/or the destruction mechanism 128. Examples of information that the sensor 118 can sense include environmental conditions (e.g., temperature, humidity, geographic location, time, etc.) and destruction mechanism status (e.g., whether or not the destruction mechanism 128 has been activated). The drug delivery device 100 can also include a communications interface 120 configured to communicate externally data which has been gathered by the sensor 118 about the drug delivery device 100, the drug contained in the drug holder 102, and/or the destruction mechanism 128, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or and to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

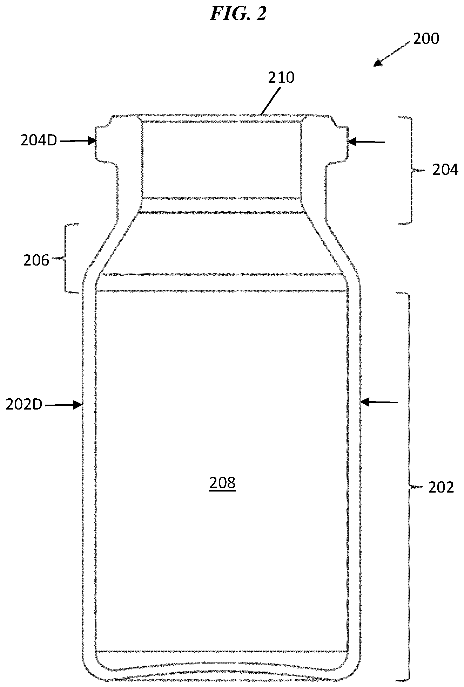

FIG. 2 illustrates an exemplary embodiment of the drug holder 102 in the form of a vial 200 that is configured to contain a drug 202 therein. The vial 200 includes a base or distal portion 202 and a head or proximal portion 204. As shown, the vial 200 also includes an inwardly tapering neck portion 206 that extends between the base portion 202 and the head portion 204. The inwardly tapering neck portion 206 allows the head portion 204 to have a maximum outer diameter 204D that is less than a maximum outer diameter 202D of the base portion 202. In other embodiments, the taper between the base and head portions 202, 204 can be omitted, and the base and head portions 302, 304 can have a same maximum outer diameter 202D, 204D as one another.

The base portion 202 defines a hollow interior 208 within the base portion 202 that is configured to contain the drug therein. In an exemplary embodiment, an amount of the drug in the vial 200 is such that the drug can be contained entirely within the hollow interior 208 of the base portion 202, but the amount of the drug can be such that drug entirely fills the hollow interior 208 of the base portion 202 and also fills at least some part of the neck portion 206 and optionally also at least some part of the head portion 204. While the base portion 202 can have a variety of configurations, in this illustrated embodiment, the base portion 202 has a substantially cylindrical shape. In other embodiments, the base portion 202 can have any other suitable shapes, e.g., a substantially rectangular shape, etc. A person skilled in the art will appreciate that a shape may not be a precise shape (e.g., a precise cylinder or a precise rectangle) but nevertheless be considered to be substantially that shape due to any number of factors, such as manufacturing tolerances and sensitivity of measurement equipment.

The vial 200 includes a seal member 210 at a proximal end thereof in the head portion 204. The seal member 210 is configured to provide a fluid tight seal such that the drug is contained in the vial 200 until the seal provided by the seal member 210 is broken. The seal provided by the seal member 210 can be broken in a variety of ways, such as by being pierced by a needle, pin, piston, etc. of the drug delivery device to which the vial 200 is releasably coupled. The seal member 210 can have a variety of configurations, as will be appreciated by a person skilled in the art, such as by being a pierceable polymer septum or a foil layer. The seal member 210 can be protected from accidental puncturing or piercing before intended use with a removable protective member or stopper, such as a tamper evident (TE) seal, etc. In some embodiments, the seal member 210 can be omitted and instead a removable protective member or stopper can be provided that is removed just prior to use of the vial 200.

An exemplary vial can include a variety of features to facilitate sealing and storing a drug therein, as described herein and illustrated in the drawings. However, a person skilled in the art will appreciate that the vials can include only some of these features and/or can include a variety of other features known in the art. The vials described herein are merely intended to represent certain exemplary embodiments.

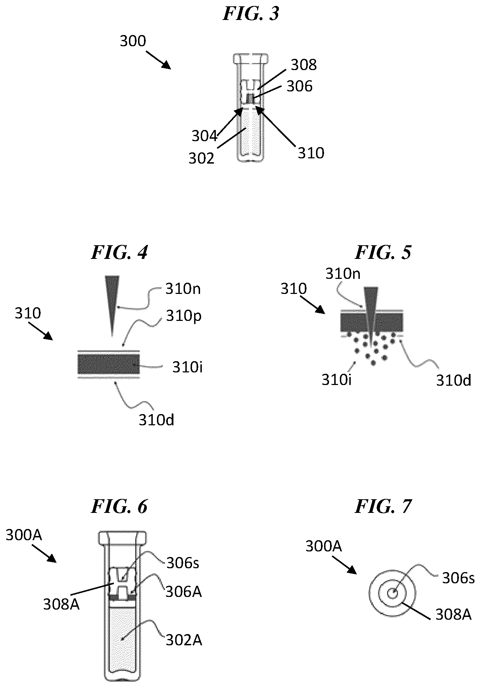

FIG. 3 illustrates an exemplary embodiment of the vial 200 that can be used as the drug holder 102 of FIG. 1 in an embodiment of the invention. In the embodiment of FIG. 3, a vial 300 is configured to contain a drug 302 in a hollow interior 304 thereof. FIG. 3 also illustrates an exemplary embodiment of the destruction mechanism 128 in the form of a chemical 306 embedded in a removable protective stopper 308 that is configured to be removed just prior to use of the vial 300. In this illustrated embodiment, the destruction mechanism 306 is configured to be activated to destroy the drug 302 in response to occurrence of a predetermined trigger event. The destruction mechanism 306 is thus configured to destroy the 302 drug contained in the drug holder 300 before actuation of an actuator of the drug delivery device configured to deliver the drug 302 from the vial 300 to a patient since the stopper 308 with the chemical 306 embedded therein will be removed just prior to use of the vial 300. A user of the vial 300 can be instructed to remove the stopper 308 just before use of the vial 300 in any of a variety of ways, such as by being verbally instructed by a medical care provider, by the instruction being included in the drug delivery device's Instructions For Use (IFU), etc.

The chemical 306 can be any of a variety of chemicals and can be different for different drugs. For example, the chemical 306 can include silica. The drug in the vial 300 is a liquid drug. Silica is configured to absorb any liquid drug containing water and thereby destroy the drug and prevent its delivery to a patient, as silica is configured to adsorb water and hold water vapor. Water molecules adhere to the surface of the silica grain. Silica gel, for example, can absorb about 40% of its weight in water. Some silica has a toxic coating of cobalt chloride, therefore silica used as the chemical 306 should be cobalt chloride free.

The vial 300 includes a barrier 310 located between the destruction mechanism 306 and the drug 302. The barrier 310 is configured to prevent the destruction mechanism 306 and the drug 302 from coming into contact with one another before occurrence of the predetermined trigger event. The barrier 310 is configured to provide a fluid tight seal between the chemical 306 and the drug such that the chemical 306 and the drug cannot contact one another until the seal provided by the barrier 310 is broken. The barrier 310 can have a variety of configurations.

FIGS. 4 and 5 illustrate an exemplary embodiment of the barrier 310. In this illustrated embodiment, the barrier 310 includes a proximal layer 310p, e.g., foil, a distal layer 310d, e.g., tensioned rubber, and an intermediate layer 310i, e.g., silica gel, that is positioned between the proximal and distal layers 310p, 310d. FIG. 4 illustrates the barrier 310 with the barrier intact 310 and not broken. FIG. 5 illustrates the barrier 310 being broken by a pin 310n, although another piercing element can be used as discussed above. The pin 30n first passes through and breaks the proximal layer 310p, then passes through the intermediate layer 310i, and then passes through and breaks the distal layer 310d. The distal layer 310d being broken allows the intermediate layer 310i to interact with the underlying drug 302. The breakage of the distal layer 310d causes the tensioned rubber to shrink outwardly to the sides of the barrier 310, which may facilitate movement of the intermediate layer 310i distally toward the drug 302 due to gravity.

Referring again to FIG. 3, instead of being embedded in the stopper 308, the chemical 306 can be in a puck, pouch, or pod attached to a distal end of the stopper 308. The barrier 310 can serve as a distal side of the puck, pouch, or pod. In this way, when the barrier 310 breaks, the chemical 306 is released to come into contact with the drug, which is located distal to the stopper 308, the chemical 306, and the barrier 310 prior to barrier 310 breakage. Examples of the chemical 306 that can be disposed in the puck, pouch, or pod include silica, acid, bleach, glycerol, sodium polyacrylate, activated carbon, zeolytes, and charcoal. As mentioned above, silica is configured to absorb liquid drug and thereby destroy the drug and prevent its delivery to a patient. Acid and bleach are configured to mix with liquid drug and render the drug ineffective. Charcoal is configured to mix with liquid drug and, should the drug be delivered into a patient, render the drug unable to be absorbed by the patient since charcoal cannot be absorbed and will pass with the drug through the patient without harm.

In some embodiments, instead of the destruction mechanism 306 being a chemical in a puck, pouch, or pod attached to a distal end of the stopper 308, the destruction mechanism can be a sponge or other absorbable material that is configured to absorb the drug therein and that is attached to a distal end of the stopper 308. In this way, similar to that discussed above, when the barrier 310 breaks, the destruction mechanism 306 and the drug no longer prevented from coming into contact with one another such that the drug can be substantially all destroyed by the sponge or other absorbable material 306 by being absorbed thereby. The destruction mechanism as a sponge or other absorbable material is discussed further below.

Occurrence of the predetermined trigger event is configured to automatically break the barrier 310 and thereby allow the destruction mechanism 306 and the drug 302 to come into contact with one another such that the destruction mechanism 306 is allowed to destroy the drug 302. A processor is operatively coupled to the barrier 310 and is configured to cause the barrier 310 to break in response to the occurrence of the predetermined trigger event.

In some embodiments, the processor configured to cause barrier 310 breakage is a processor of the drug delivery device that includes the vial 300, e.g., the processor 122 of the drug delivery device 100. In such embodiments, the processor is operatively coupled to a sensor, e.g., the sensor 118 of the drug delivery device 100.

The sensor can be a location sensor configured to monitor geographic location, e.g., via satellite position determination, such as GPS. The processor can be configured to determine, based on the location data sensed by the sensor and communicated to the processor, whether the sensed geographic location is an acceptable geographic location or an unacceptable geographic location. An acceptable geographic location is a location within the drug holder's expected supply chain. The acceptable geographic locations for particular drug delivery devices will vary, as different drug delivery devices are shipped using different shipping channels based on factors such as a drug delivery device's intended end location, the manufacturer of the drug delivery device, etc. The acceptable geographic locations for a particular drug delivery device can be programmed into the drug delivery device's memory, e.g., the memory 124 of the drug delivery device 100. The processor is configured to use the stored acceptable geographic locations to determine whether or not the sensed geographic location is acceptable, e.g., by using a lookup table. If the geographic location is an acceptable geographic location, the processor is configured to take no action with respect to the barrier 310 since the drug delivery device's sensed geographic location indicates that the drug delivery device is being shipped as intended and has not been accidentally misdirected during shipping or been intercepted and transported by an unauthorized party. If the geographic location is an unacceptable geographic location, the processor is configured to cause the barrier 310 to break and, thus, for the drug to be destroyed by the destruction mechanism 306, since the drug delivery device's sensed geographic location indicates that the drug delivery device is not being shipped as intended and may have been accidentally misdirected during shipping or been intercepted and transported by an unauthorized party.

In addition to or as an alternative to the sensor including a location sensor configured to monitor location, the sensor can be configured to monitor one or more other environmental conditions, such as any one or more of a temperature sensor (e.g., a thermistor, a thermocoupler, a thermistor, etc.) configured to monitor temperature, a humidity sensor (e.g., a thermistor, a humistor, a hygrometer, etc.) configured to monitor humidity, a pH sensor configured to monitor pH level, an ultraviolet (UV) sensor configured to monitor UV exposure, and a time sensor (e.g., a timer or clock device such as an atomic clock) configured to monitor time. In embodiments in which the sensor includes a temperature sensor, the processor can be configured to determine, based on the temperature data sensed by the sensor and communicated to the processor, whether the sensed temperature is an acceptable temperature or an unacceptable temperature. An unacceptable temperature is a temperature at which intermittent and/or cumulative exposure thereto can lead to adverse effects on the potency and stability of the drug, thereby decreasing efficacy and shelf-life. In some instances, certain temperatures can ultimately render the drug non-viable for use. Acceptable temperatures for particular drugs will vary, as different drugs have different temperature needs, and in some cases, no temperature restraints at all. The acceptable temperatures for a particular drug can be programmed into the drug delivery device's memory and used by the processor similar to that discussed above regarding geographic location. If the temperature is an acceptable temperature, the processor is configured to take no action with respect to the barrier 310 since the drug delivery device's sensed temperature indicates that the drug has not experienced any adverse temperature effects. If the temperature is an unacceptable temperature, the processor is configured to cause the barrier 310 to break and, thus, for the drug to be destroyed by the destruction mechanism 306, since the drug delivery device's sensed temperature indicates that the drug has experienced an adverse temperature that may have rendered the drug ineffective and/or unsafe. The processor can be configured to cause the barrier 310 to break after only one unacceptable sensed temperature, as any adverse temperature experience for some drugs may render the drug ineffective and/or unsafe. Alternatively, the processor can be configured to cause the barrier 310 to break only after a plurality of sensed temperatures are determined by the processor to be unacceptable, thereby taking a drug's prolonged exposure to an adverse temperature into account before the drug is destroyed. Some drugs may only become ineffective and/or unsafe after experiencing too high or too low a temperature only after being subject to the too high or too low temperature for a minimum amount of time, which can be reflected by a plurality of sensed temperatures over time being too low or too high. The sensor can also include a time sensor to facilitate the processor's determination of a total length of time that the drug has been subject to an unacceptable temperature.

In embodiments in which the sensor includes a humidity sensor, the processor can be configured to determine, based on the humidity data sensed by the sensor and communicated to the processor, whether the sensed humidity is an acceptable humidity or an unacceptable humidity. Unacceptable humidity is a humidity at which intermittent and/or cumulative exposure thereto can lead to adverse effects on the potency and stability of the drug, thereby decreasing efficacy and shelf-life. In some instances, certain humidity levels can ultimately render the drug non-viable for use. Acceptable humidity for particular drugs will vary, as different drugs have different humidity needs, and in some cases, no humidity restraints at all. Acceptable humidity for a particular drug can be programmed into the drug delivery device's memory and used by the processor similar to that discussed above regarding geographic location. If the humidity is an acceptable humidity, the processor is configured to take no action with respect to the barrier 310 since the drug delivery device's sensed humidity indicates that the drug has not experienced any adverse humidity effects. If the humidity is an unacceptable humidity, the processor is configured to cause the barrier 310 to break and, thus, for the drug to be destroyed by the destruction mechanism 306, since the drug delivery device's sensed humidity indicates that the drug has experienced adverse humidity that may have rendered the drug ineffective and/or unsafe. The processor can be configured to cause the barrier 310 to break after only one unacceptable sensed humidity, as any adverse humidity experience for some drugs may render the drug ineffective and/or unsafe. Alternatively, the processor can be configured to cause the barrier 310 to break only after a plurality of sensed humidity measurements are determined by the processor to be unacceptable, thereby taking a drug's prolonged exposure to adverse humidity into account before the drug is destroyed. Some drugs may only become ineffective and/or unsafe after experiencing too high or too low a humidity only after being subject to the too high or too low humidity for a minimum amount of time, which can be reflected by a plurality of sensed humidity measurements over time being too low or too high. The sensor can also include a time sensor to facilitate the processor's determination of a total length of time that the drug has been subject to unacceptable humidity.

In embodiments in which the sensor includes a pH sensor, the processor can be configured to determine, based on the pH data sensed by the sensor and communicated to the processor, whether the sensed pH is an acceptable pH or an unacceptable pH. Unacceptable pH is a pH level at which intermittent and/or cumulative exposure thereto can lead to adverse effects on the potency and stability of the drug, thereby decreasing efficacy and shelf-life. In some instances, certain pH levels can ultimately render the drug non-viable for use. Acceptable pH for particular drugs will vary, as different drugs have different pH levels, and in some cases, no pH restraints at all. Acceptable pH for a particular drug can be programmed into the drug delivery device's memory and used by the processor similar to that discussed above regarding geographic location. If the pH is an acceptable pH, the processor is configured to take no action with respect to the barrier 310 since the drug delivery device's sensed pH indicates that the drug is at a safe and expected pH level. If the pH is an unacceptable pH, the processor is configured to cause the barrier 310 to break and, thus, for the drug to be destroyed by the destruction mechanism 306, since the drug delivery device's sensed pH indicates that the drug has a pH level that may have rendered the drug ineffective and/or unsafe. The processor can be configured to cause the barrier 310 to break after only one unacceptable sensed pH level, as any unexpected, atypical pH level for some drugs may render the drug ineffective and/or unsafe. Alternatively, the processor can be configured to cause the barrier 310 to break only after a plurality of sensed pH measurements are determined by the processor to be unacceptable, thereby taking a drug's pH level over time into account before the drug is destroyed. Some drugs may only become ineffective and/or unsafe only after having too high or too low a pH for a minimum amount of time, which can be reflected by a plurality of sensed pH measurements over time being too low or too high. The sensor can also include a time sensor to facilitate the processor's determination of a total length of time that the drug has had an unacceptable pH.

In embodiments in which the sensor includes a UV sensor, the processor can be configured to determine, based on the UV data sensed by the sensor and communicated to the processor, whether the sensed UV is an acceptable UV exposure level or an unacceptable UV exposure level. Unacceptable UV is a UV level at which intermittent and/or cumulative exposure thereto can lead to adverse effects on the potency and stability of the drug, thereby decreasing efficacy and shelf-life. In some instances, certain UV levels can ultimately render the drug non-viable for use. Acceptable UV for particular drugs will vary, as different drugs have different UV reactions, and in some cases, no UV reactions at all. Acceptable UV for a particular drug can be programmed into the drug delivery device's memory and used by the processor similar to that discussed above regarding geographic location. If the UV exposure level is an acceptable UV exposure level, the processor is configured to take no action with respect to the barrier 310 since the drug delivery device's sensed humidity indicates that the drug has not experienced any adverse UV effects. If the humidity is an unacceptable UV exposure level, the processor is configured to cause the barrier 310 to break and, thus, for the drug to be destroyed by the destruction mechanism 306, since the drug delivery device's sensed UV level indicates that the drug has had adverse UV exposure that may have rendered the drug ineffective and/or unsafe. The processor can be configured to cause the barrier 310 to break after only one unacceptable sensed UV level, as any adverse UV experience for some drugs may render the drug ineffective and/or unsafe. Alternatively, the processor can be configured to cause the barrier 310 to break only after a plurality of sensed UV measurements are determined by the processor to be unacceptable, thereby taking a drug's prolonged exposure to adverse UV into account before the drug is destroyed. Some drugs may only become ineffective and/or unsafe after being exposed to too high or too low a UV level only after being exposed to the too high or too low UV level for a minimum amount of time, which can be reflected by a plurality of sensed UV measurements over time being too low or too high. The sensor can also include a time sensor to facilitate the processor's determination of a total length of time that the drug has been exposed to unacceptable UV.

Regardless of a type of the sensor, once the processor has caused the barrier 310 to break or otherwise prevent drug access, the processor can be configured to cause the sensor to cease gathering data since the drug has already been destroyed or rendered unable to be delivered from the drug delivery device.

In embodiments in which the drug is esketamine, ketamine, or another controlled substance, and the drug is in the United States, a Drug Enforcement Administration (DEA) Registration Number is assigned to one or more health care providers authorized to prescribe the drug. In such embodiments, the DEA Registration Number can act as an electronic key used by the processor to determine whether or not to cause destruction of the drug. The processor can be configured to determine whether a shipment including the drug delivery device is received by an acceptable DEA Registration Number, e.g., one of the assigned DEA Registration Numbers for the drug in the vial 300. Acceptable DEA Registration Numbers for a particular drug can be programmed into a memory accessible by the processor and used by the processor similar to that discussed above regarding geographic location. If the DEA Registration Number is an acceptable DEA Registration Number, the processor is configured to take no action with respect to the barrier 310 since the DEA Registration Number indicates that an authorized party has received the drug. If the DEA Registration Number is an unacceptable DEA Registration Number, e.g., is not among the assigned DEA Registration Numbers for the drug, or if a DEA Registration Number is absent, the processor is configured to cause the barrier 310 to break and, thus, for the drug to be destroyed by the destruction mechanism 306, since the drug is presumed to be with an unauthorized party. The processor can be configured to verify the DEA Registration Number as an acceptable DEA Registration Number by communicating with, e.g., via a communications interface and/or using an app, an external device that maintains a database of acceptable DEA Registration Numbers.

In other embodiments, the processor configured to cause barrier 310 breakage is external to and separate from the drug delivery device that includes the vial 300. In such embodiments, the processor is operatively coupled to a sensor. The sensor is configured similar to that discussed above regarding embodiments in which the processor is a processor of the drug delivery device that includes the vial 300, e.g., includes any one or more of a location sensor, a temperature sensor, a humidity sensor, a pH sensor, a UV sensor, and a time sensor. The processor that is external to and separate from the drug delivery device that includes the vial 300 is operatively coupled to a communications interface that is also external to and separate from the drug delivery device and that is configured to communicate with a communications interface of the drug delivery device, e.g., the communications interface 120 of the drug delivery device 100. In response to determining that drug destruction is required based on the sensed data and/or an unacceptable or absent DEA Registration Number, as discussed above, the external, separate processor is configured to cause the external, separate communications interface to communicate an instruction to the drug delivery device, via the drug delivery device's communications interface, to cause barrier 310 breakage. A processor of the drug delivery device that includes the vial 300, e.g., the processor 122 of the drug delivery device 100, is configured to execute the instruction similar to that discussed above regarding the on-board processor causing barrier 310 breakage. Regardless of a type of the sensor, once the processor has caused the barrier 310 to break or otherwise prevent drug access, the processor can be configured to cause the sensor to cease gathering data since the drug has already been destroyed or rendered unable to be delivered from the drug delivery device.

FIGS. 6 and 7 illustrate another embodiment of a drug delivery device 300A configured to expel a drug into a nose of a patient and configured to destroy drug on board the drug delivery device 300A using an exemplary embodiment of the destruction mechanism 128 in the form of a chemical 306A configured to destroy a drug 302A contained in the vial 300A. The drug delivery device 300A is generally configured and used similar to the drug delivery device 300 of FIG. 3 except that the drug delivery device 300A includes a stopper 308A as a seal member that need not be removed just prior to use of the vial 300A. Instead, a piston, pin, and/or a needle can be configured to pierce through or puncture the stopper 308A, e.g., through a septum 308s thereof. The chemical 306A is a ring form factor or otherwise located relative to the septum 308s to allow the a piston, pin, and/or a needle to pierce through or puncture the stopper 308A without piercing through or puncturing the chemical 306A.

In some embodiments, instead of a destruction mechanism being located proximal to a drug in a vial, the destruction mechanism can be located distal to the drug in the vial. A stopper in the vial can thus be an existing stopper and need not have the destruction mechanism coupled thereto or otherwise be modified form its existing design and use.

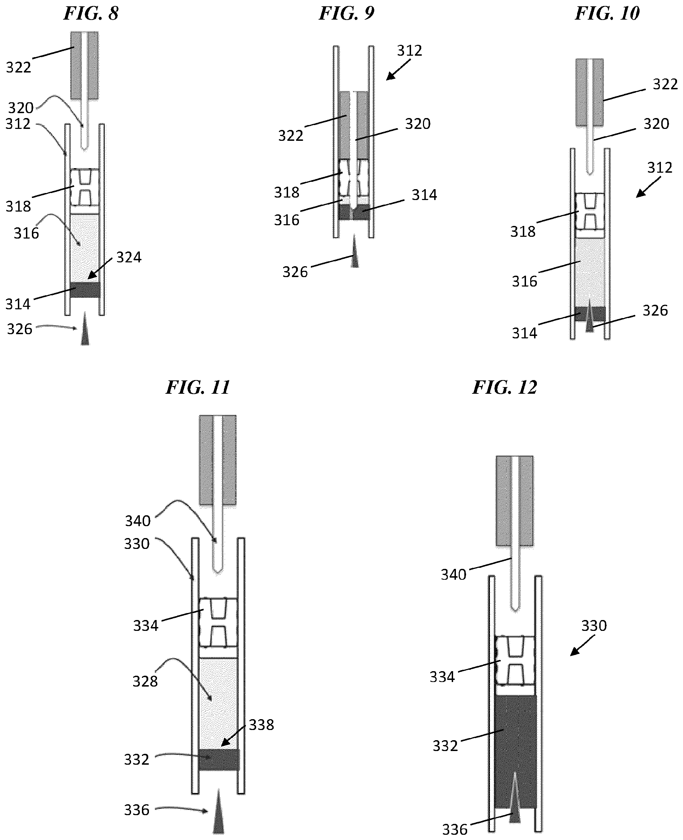

FIG. 8 illustrates another embodiment, according to the invention, of a drug delivery device configured to expel a drug into a nose of a patient and configured to destroy drug on board the drug delivery device using an exemplary embodiment of the destruction mechanism 128 in the form of a chemical 314 configured to destroy a drug 316 contained in a vial 312. The drug delivery device is generally configured and used similar to the drug delivery device 300 of FIG. 3 except that the vial 312 in this illustrated embodiment is an extruded vial, and the destruction mechanism 314 is located distal to the drug 316 in the vial 312. The destruction mechanism 314 is thus also located distal to a stopper 318 in the vial 312. As discussed above, the stopper 318 is configured to be pierced or punctured by a piercing element, which is a needle 320 attached to a cannula 322 in this illustrated embodiment.

The vial 312 in this illustrated embodiment is an extruded vial that is formed of an extruded piece of glass with the stopper 318 defining a seal member at a proximal end of the vial 312 and a barrier 324 defining a seal member at a distal end of the vial 312. The barrier 324 is disposed between the drug 316 and the destruction mechanism 314. The stopper 318 and the barrier 324 are thus configured to cooperate to contain the drug 316 in the vial 312 until the drug 316 is destroyed or delivered out of the drug delivery device's opening. Forming the vial 312 via extrusion may be a less expensive method of manufacturing a vial than other manufacturing methods such as molding and/or may facilitate recycling of the vial 312 and/or disposal of the vial 312 as medical waste by facilitating separation of the vial 312 from a remainder of the drug delivery device, which itself may be reused with another vial, may be recycled, or may be disposed of as medical waste.

FIG. 8 illustrates the vial 312 before delivery of the drug 316 from the drug delivery device and before the chemical 314 has destroyed any of the drug 316. FIG. 9 illustrates the vial 312 after delivery of the drug 316 from the drug delivery device with the needle 320 having pierced or punctured the stopper 318. A residual amount of the drug 316 remains in the vial 312. The needle 320 has penetrated through the barrier 324, thereby allowing the chemical 314 and the drug 316 to come into contact with one another to destroy substantially all of the residual drug 316 remaining in the vial 312.

FIG. 10 illustrates the vial 312 before delivery of the drug 316 from the drug delivery device and with a pin 326 breaking the barrier 324, although as discussed above another piercing element can be used. The pin 326 first passes through the destruction mechanism 314 and then through the barrier 324 to allow the chemical 314 and the drug 316 to come into contact with one another to destroy substantially all of the drug 316 in the vial 312 prior to drug delivery.

FIG. 11 illustrates another embodiment, according to the invention, of a drug delivery device configured to expel a drug into a nose of a patient and configured to destroy drug on board the drug delivery device using an exemplary embodiment of the destruction mechanism 128 configured to destroy a drug 328 contained in a vial 330. The drug delivery device is generally configured and used similar to the drug delivery device of FIG. 8 except that in this illustrated embodiment, the destruction mechanism 332, which is located distal to the drug 328 in the vial 330 and is located distal to a stopper 334 in the vial 330, is in the form of a sponge, although as mentioned above another absorbable material can be used. FIG. 11 illustrates the vial 330 before delivery of the drug 328 from the drug delivery device and before the destruction mechanism 332 has absorbed any of the drug 328. FIG. 12 illustrates the vial 330 before delivery of the drug 328 from the drug delivery device and with a pin 336 breaking a barrier 338 disposed between the drug 328 and the destruction mechanism 332, although as discussed above another piercing element can be used. The pin 336 first passes through the destruction mechanism 332 and then through the barrier 338 to allow the destruction mechanism 332 and the drug 328 to come into contact with one another to destroy substantially all of the drug 328 in the vial 330 prior to drug delivery. As shown in FIG. 12, the absorption of the drug 328 causes the sponge 332 to move from a compressed configuration, shown in FIG. 11, to an expanded configuration in which the sponge 332 is expanded in the vial 330. The destruction mechanism 332 can instead destroy substantially all residual drug in the vial 330 after drug delivery by a needle 340 (or other piercing element) piercing or puncturing the barrier 338 and the destruction mechanism 332, similar to that discussed above regarding the vial 312 and the destruction mechanism 314 of FIG. 9.

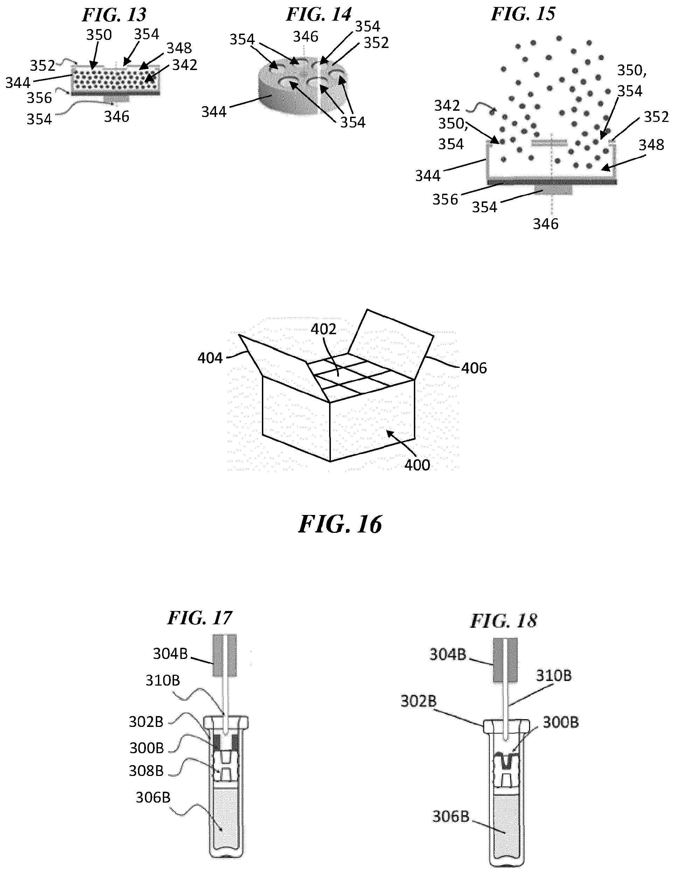

FIGS. 13 and 14 illustrate another exemplary embodiment of the destruction mechanism 128 that can be used with a drug delivery device configured to expel a drug into a nose of a patient. The drug delivery device is generally configured and used similar to the drug delivery device of FIG. 8 except that in this illustrated embodiment, the destruction mechanism 342, which is located distal to the drug in the drug delivery device's drug holder, e.g., an extruded vial, in the form of a chemical, e.g., balls of silica or other substance, is configured to be electrically driven into contact with the drug. A housing 344 contains the chemical 342 in an inner cavity 348 thereof and includes a hole 350 in a proximal end thereof. A cover 352 is positioned over the proximal end of the inner cavity 348. The cover 352 includes a hole 354 formed therein. The cover 352 includes six holes 354 in this illustrated embodiment but can include another number, e.g., one, two, three, four, etc. In an exemplary embodiment the housing 344 has a same number of holes 350 as the cover 352, as in this illustrated embodiment.

The cover 352 is configured to rotate about a longitudinal axis 346 of the cover 352 relative to the housing 344. The longitudinal axis 346 of the cover 352 also defines a longitudinal axis of the housing 344. A motor 354 is configured to drive the rotation of the cover 352. A power source 356, e.g., a coin cell battery or other power source, is configured to provide power to the motor 354.

In an initial configuration of the destruction mechanism 342, which is shown in FIGS. 13 and 14, the holes 350 of the housing 344 are misaligned from the holes 354 of the cover 352. The drug 342 is thus prevented from escaping the housing 344 through any of the holes 350, 354. The chemical 342 thus cannot contact the drug in the drug holder. The rotation of the cover 352 is configured to cause the holes 350 of the housing 344 to become aligned with the holes 354 of the cover 352 such that the chemical 342 is allowed to escape from the housing 344 through the holes 350, 354, as shown in FIG. 15. The chemical 342 can thus come into contact with the drug in the drug holder and destroy the drug.

In an exemplary embodiment, a box or other package containing the drug delivery device therein includes the sensor and the external, separate processor and communications interface. The box or other package can include only the one drug delivery device therein or can include therein a plurality of drug delivery devices each including a vial therein, e.g., the vial 300 of FIG. 3, the vial 300A of FIG. 6, the vial 312 of FIG. 8, the vial 330 of FIG. 11, the vial 302B of FIG. 17, etc.

FIG. 16 illustrates one embodiment of a box 400 configured to contain therein a plurality of drug delivery devices each including a vial 300. The box 400 in this illustrated embodiment is a shipping box in which the drug delivery devices are configured to be shipped along a supply chain. The box 400 includes an electronics package 402, e.g., a chip, circuit board, etc., that includes the processor, the communications interface, and the sensor. In an exemplary embodiment, the electronics package 402 is located inside the box 400, e.g., attached to an internal surface thereof, such that the electronics package 402 is inaccessible when the box 400 is closed, which may help prevent damage to and/or tampering with the electronics package 402 during the shipping process. The box 400 is shown open in FIG. 16 for clarity of illustration, but the box 400 would be closed during shipping.

The electronics package 402 can include a switch configured to be closed when the box 400 is closed and to be open when the box 400 is open. For example, the switch can be positioned across a seam of the box 400 defined by flaps 404, 406 of the box 400. The seam is closed when the box 400 is closed, and the seam is open when the box 400 is open. When the switch is closed, the sensor is configured to be in an active state in which the sensor gathers data that can be used by the processor to trigger drug destruction if determined to be necessary. When the switch is open, the sensor is configured to be in an inactive state in which the sensor cannot gather data such that, if drug destruction has not yet been triggered by the processor, drug destruction can no longer be triggered. The box 400 being opened indicates that the shipping process has been completed as intended.

In another exemplary embodiment in which the processor configured to cause barrier 310 breakage is external to and separate from the drug delivery device that includes the vial 300, radio frequency identification (RFID) is used in determining whether or not to destroy the drug. In such embodiments, a box or other package containing the drug delivery device therein, e.g., similar to the box 400 of FIG. 16, includes an RFID tag configured to be read by an external scanning device, e.g., an RFID reader. In an exemplary embodiment, the RFID tag is located inside the box, e.g., attached to an internal surface thereof, such that the RFID tag is inaccessible when the box is closed, which may help prevent damage to and/or tampering with the RFID tag during the shipping process. The RFID reader includes the external, separate processor, or another device configured to electronically receive data from the RFID reader, e.g., scanned RFID tag data, includes the external, separate processor. The RFID reader can be handheld and configured to be manually operated to read the RFID tag, or the RFID reader can be mounted in a facility, e.g., on a ceiling, on a wall, etc., and be configured to automatically read the RFID tag when in effective distance of the RFID tag.

Similar to the DEA Registration Number discussed above, the RFID tag can act as an electronic key used by the processor to determine whether or not to cause destruction of the drug. The processor can be configured to determine whether a shipment including the drug delivery device is received by an acceptable party, e.g., one of the known parties in the supply chain. Acceptable identification codes for a particular drug and/or particular drug delivery device can be programmed into a memory accessible by the processor and used by the processor similar to that discussed above regarding geographic location. If the identification code in the scanned RFID tag data is an acceptable identification code, the processor is configured to take no action with respect to the barrier since the identification code indicates that an authorized party has received the drug. If the identification code is an unacceptable identification code, e.g., is not among the stored identification codes, or if an identification code is absent from the scanned RFID tag data, the processor is configured to cause the barrier to break and, thus, for the drug to be destroyed by the destruction mechanism, since the drug is presumed to be with an unauthorized party.

In an exemplary embodiment in which an RFID tag is used, the processor is operatively coupled to a time sensor that the processor is configured to also use in determining whether or not to cause drug destruction. The time sensor can act as a timer that the processor uses in connection with scanned RFID tag data. If scanned RFID tag data is not received within a predetermined amount of time that has elapsed since a start time, e.g., a time that the box or other package including the RFID tag leaves the immediately prior party in the supply chain or a time that the immediately prior RFID tag scan occurred, the processor can be configured to cause drug destruction since the box or other package is presumed to have not reached its next intended destination in the supply chain since no RFID tag was scanned within the predetermined time period.

In some embodiments, each scan of the RFID tag creates a new tag to be included in at least the next scan of the RFID tag if not in all subsequent scans of the RFID tag. In this way, the processor can verify that the supply chain has remained intact as expected by verifying with each scan that each tag in the chain is acceptable and, if not, cause drug destruction.

Instead of an RFID tag, another type of tag can be similarly used. For example, a box or other package containing the drug delivery device therein can also include therein a Tile^{™} device, available from Tile Inc. of San Mateo, CA. A computer device, such as a mobile phone, an electronic tablet, etc., with the Tile App installed therein is configured to use the Tile App to "discover" the Tile^{™} device via Bluetooth communication. The computer device can include the external, separate processor, or another device configured to electronically receive data, e.g., the Bluetooth received data, from the computer device that includes the external, separate processor. Examples of tags other than the Tile^{™} device may be used.

Referring again to FIG. 1, in another exemplary embodiment, the destruction mechanism 128 includes a heating element configured to heat the drug in the drug holder 102 and thereby destroy the drug. The heating element can have any of a variety of configurations, such as a heating coil, a heating cable, a positive temperature coefficient (PTC) heater, or a resistive element configured to warm as current passes therethrough.

A processor, either the processor 122 of the drug delivery device 100 or a processor external to and separate from the drug delivery device 100 as discussed above, is configured to turn the heating element on (providing heat) and off (not providing heat). The processor can be configured to turn on the heating element for a predetermined amount of time, e.g., an amount stored in a memory accessible to the processor, e.g., the memory 124 of the drug delivery device 100 or a memory external to and separate from the drug delivery device 100, before causing the heating element to turn off. The predetermined amount of time can be based on one or more factors, such as on a type of the drug and/or on a volume of drug in the drug holder 102. The heating element being on for the predetermined amount of time limits heating of the drug by the heating element, which may help ensure that the drug is destroyed but that the drug delivery device 100 as a whole and/or that one or more components thereof do not become too hot to touch and/or become a fire hazard.

Instead of or in addition to the processor being configured to turn the heating element off based on an elapse of a predetermined amount of time since the heating element was turned on, the processor can be configured to turn on the heating element until the drug reaches a predetermined maximum temperature, as sensed by a temperature sensor configured to communicate gathered temperature data to the processor, at which time the processor can be configured to turn off the heating element. The predetermined maximum temperature can be stored in a memory accessible to the processor, e.g., the memory 124 of the drug delivery device 100 or another memory. The predetermined maximum temperature can vary based on the drug, as different drugs can become ineffective at different maximum temperatures.

The heating element can be at a variety of locations relative to the drug holder 102. For example, the heating element can be located at least partially within the drug holder 102, such as by being wrapped or coiled around an interior surface of the drug holder 102, being located on a bottom interior surface of the drug holder 102, extending through a fluid-tight opening formed through a sidewall or other surface of the drug holder 102, etc. The heating element may therefore be in direct contact with the drug in the drug holder 102, which may speed warming of the drug by the heating element as compared to embodiments in which the heating element is not in direct contact with the drug, e.g., by the heating element being located entirely outside of the drug holder 102.

In some embodiments, the destruction mechanism 128 includes a heating element in addition to a substance configured to be heated by the heating element, to decompose when heated by the heating element, and to destroy the drug in its decomposed state. Such embodiments are a hybrid of the above-discussed embodiments in which the destruction mechanism 128 includes a substance configured to destroy the drug when the substance comes in contact with the drug and the above-discussed embodiments in which the destruction mechanism 128 includes a heating element.

The substance configured to be heated by the heating element, to decompose when heated by the heating element, and to destroy the drug in its decomposed state can have a variety of configurations. For example, the substance can include a polymer or a wax attached to a distal end of a stopper at a proximal end of the drug holder 102. The heating of the polymer or wax by the heating element can cause the polymer or wax to change state from a first state to a second state in which the polymer or wax is configured to destroy the drug, similar to that discussed above. For another example, the substance can include a polymer or wax lining on at least some of an interior surface of the drug holder 102 that is in contact with the drug. The heating of the polymer or wax by the heating element can cause the polymer or wax to change state from a first state to a second state in which the polymer or wax is configured to destroy the drug, similar to that discussed above. For yet another example, the polymer or wax can encapsulate a chemical, as discussed above. The heating of the polymer or wax by the heating element can cause the polymer or wax to degrade or melt and thereby allow the chemical to encounter and destroy the drug.

Regardless of whether the heating element is configured to heat the drug directly to destroy the drug or to heat a substance that destroys or prevents access to the drug, the processor can be configured to determine, based on data sensed by a sensor and communicated to the processor, whether the heating element should be turned on. The sensed data can be any of the data discussed above, e.g., sensed geographic location to indicate an acceptable geographic location or an unacceptable geographic location, sensed temperature to indicate an acceptable temperature or an unacceptable temperature, sensed humidity to indicate an acceptable humidity or an unacceptable humidity, sensed pH to indicate an acceptable pH level or an unacceptable pH level, and/or sensed UV to indicate an acceptable UV level or an unacceptable UV level.

Regardless of a type of the sensor, once the processor has caused the heating element to turn on and thereby destroy or prevent access to the drug, the processor can be configured to cause the sensor to cease gathering data since the drug has already been destroyed or rendered unable to be delivered from the drug delivery device.

In addition to or instead of the processor being configured to activate the heating element in response to data gathered by the sensor, the processor can be configured to turn on the heating element in response to other criteria similar to that discussed above, such as DEA Registration Number, RFID tag data, Tile^{™} data, or other tag data.

Regardless of whether the heating element is configured to heat the drug directly to destroy the drug or to heat a substance that destroys the drug, the processor can be configured to turn the heating element on before actuation of the actuator 106 of the drug delivery device 100 so as to destroy or prevent access to substantially all of the drug in the drug holder 102. Alternatively or in addition, the processor can be configured to turn the heating element on after an actuation of the actuator 106 of the drug delivery device 100 so as to destroy or prevent access to substantially all residual drug left in the drug holder 102 after completion of drug delivery. In such embodiments, the processor can be configured to turn the heating element on automatically after the actuator 106 has been actuated once to help ensure destruction of substantially all residual drug left in the drug holder 102 after completion of drug delivery. The processor can also be configured to turn the heating element off, as discussed above. For example, the processor can be configured to turn the heating element on after a predetermined amount of time has elapsed since the actuation of the actuator 106. As discussed above, elapsed time can be monitored by a time sensor operatively coupled to the processor.

FIG. 17 illustrates another exemplary embodiment of the destruction mechanism 128 that can be used with a drug delivery device configured to expel a drug into a nose of a patient. The drug delivery device is generally configured and used similar to the drug delivery device 100 of FIG. 1. In this illustrated embodiment, the drug delivery device's destruction mechanism includes a heating element and a substance 300B configured to be heated by the heating element to prevent a drug 306B from being delivered from a vial 302B and out of the drug delivery device's opening. The vial 302B is generally configured and used similar to the vial 200 of FIG. 2. The substance 300B is located proximal to a stopper 308B in the vial 302B, and thus proximal to the drug in the vial 302B, and is located distal to the drug delivery device's cannula 304B and thus distal to the opening.

In an unheated configuration, shown in FIG. 17, the destruction mechanism 300B has a ring shape with a central opening. The central opening allows a needle 310B attached to the cannula 304B to pass therethrough to access and pierce or puncture the stopper 308B. The drug 306B thus can be delivered from the vial 300B and out of the drug delivery device with the destruction mechanism 300B in the unheated configuration. In a heated configuration, shown in FIG. 18, the destruction mechanism 300B provides a barricade that prevents the needle 310B from accessing the stopper 308B and thus from piercing or puncturing the stopper 308B. The drug 306B thus cannot be delivered from the vial 300B and out of the drug delivery device with the destruction mechanism 300B in the heated configuration.

In response to occurrence of a predetermined event, as discussed above, the heating element is configured to cause the destruction mechanism 302B to move from the unheated configuration to the heated configuration. The heating element is configured to heat the substance 300B and thereby cause the substance 300B to melt. The melting of the substance 300B causes the substance 300B to lose its ring shape and ooze over the stopper 308B to provide the barricade. The substance 300B can have a variety of configurations, such as a wax or a low melting temperature polymer. Examples of polymers with a low melting temperature include polycaprolactone (PCL) thermoplastic with a melting temperature of about 60°C melting and Polyvinyl Chloride (PCV) with a melting temperature of about 100°C. A person skilled in the art will appreciate that a value may not be precisely at a value but nevertheless be considered to be at about that value due to any number of factors, such as manufacturing tolerances and sensitivity of measurement equipment.

Referring still to FIG. 1, regardless of the particular configuration of the destruction mechanism 128, the processor (either the processor 122 of the drug delivery device 100 or a processor external to and separate from the drug delivery device 100) can be configured to prevent drug delivery from the drug delivery device 100 in addition to causing the destruction mechanism 128 to destroy the drug. In this way, the drug cannot be delivered to a patient from the drug delivery device after a determination that the drug should be destroyed, which may help prevent the drug from being delivered from the drug delivery device to an unauthorized user, may prevent an unsafe and/or ineffective drug from being delivered to a patient and bringing with delivery a false belief that a safe, effective drug dose was delivered, and/or may prevent any of the destruction mechanism 128 as a chemical or other substance from being delivered through the opening 108 to the patient. The processor can be configured to prevent drug delivery from the drug delivery device 100 in any of a variety of ways. For example, the processor can be configured to disable the device's power supply 126 in embodiments in which the actuator 106 requires power from the power supply 126 to be actuated, such as by opening a switch in a circuit that operatively connects the power supply 126 and the actuator 106. For another example, the processor can be configured to activate a device operation prevention mechanism of the drug delivery device 100 which prevents dispensing of the drug by, for example, preventing accidental movement of the dispensing mechanism 104 and/or accidental actuation of the actuator 106.

Referring still to FIG. 1, regardless of the particular configuration of the destruction mechanism 128, the predetermined trigger event, the occurrence of which triggers the processor (either the processor 122 of the drug delivery device 100 or a processor external to and separate from the drug delivery device 100) to cause the destruction mechanism 128 to destroy the drug, can be an instruction received by the processor to cause drug destruction. The processor can be operatively coupled to a communications interface (either the communications interface 120 of the drug delivery device 100 for the processor 122 or a communications interface external to and separate from the drug delivery device 100 for a processor external to and separate from the drug delivery device 100) that receives the instruction. The instruction can be transmitted from any of a variety of computer systems configured to transmit such an instruction, e.g., a desktop computer, a laptop computer, a mobile phone, an electronic tablet, etc. In an exemplary embodiment, the computer system transmits the instruction in response to an input to the computer system from a health care provider of the patient with which the drug delivery device 100 is associated.

Referring still to FIG. 1, regardless of the particular configuration of the destruction mechanism 128, in embodiments in which the actuator 106 is configured to be actuated a plurality of times to deliver a plurality of doses of the drug from the drug holder 102, the predetermined trigger event, the occurrence of which triggers the processor (either the processor 122 of the drug delivery device 100 or a processor external to and separate from the drug delivery device 100) to cause the destruction mechanism 128 to destroy the drug, can be a predetermined number of actuations of the actuator 106. In this way, the destruction mechanism 128 can be configured to destroy any residual drug left in the drug holder 102 after completion of all of the drug deliveries causes by the multiple actuations of the actuator 106. The predetermined number of actuations of the actuator 106 can be any plural number, e.g., two, three, four, five, six, seven, etc., depending on factors such as a total amount of the drug in the drug holder 102 before a first actuation of the actuator 106, a size of each drug dose, and a type of the drug. The predetermined number of actuations of the actuator 106 can be stored in a memory accessible to the processor, e.g., the memory 124 of the drug delivery device 100 or a memory external to and separate from the drug delivery device 100.

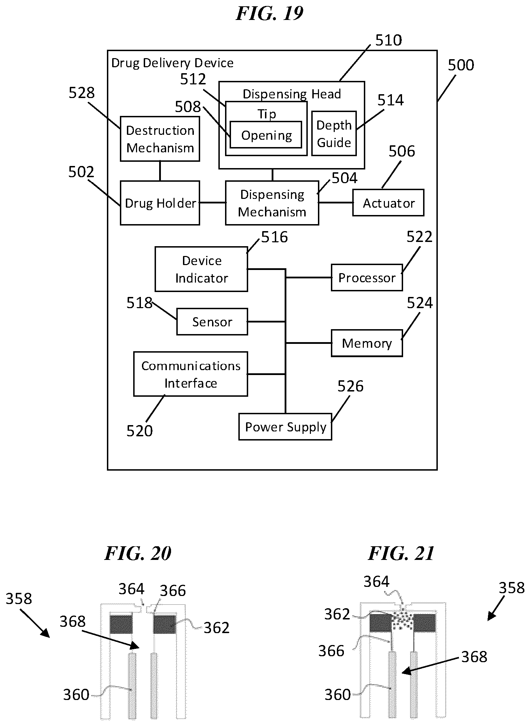

FIG. 19 illustrates another embodiment of a drug delivery device 500 configured to expel a drug into a nose of a patient and configured to destroy drug on board the drug delivery device 500 using a destruction mechanism 528. The drug delivery device 500 of FIG. 19 is generally configured and used similar to the drug delivery device 100 of FIG. 1 and includes a drug holder 502, a dispensing mechanism 504, an actuator 506, an opening 508, a tip 510, a dispensing head 512, a depth guide 514, a device indicator 516, a sensor 518, a communications interface 520, a processor 522, a memory 524, a power supply 526, and a destruction mechanism 528. However, in the illustrated embodiment of FIG. 19, the drug holder 502 does not include the destruction mechanism 528. Instead, the destruction mechanism 528 is located external to the drug holder 102 and is configured to operatively engage the drug in the drug holder 502 to destroy the drug. For example, in an embodiment according to the invention, the destruction mechanism 528 includes a heating element, as discussed above, that is wrapped or coiled around the drug holder 502, e.g., wrapped or coiled one or more times around an exterior perimeter of the drug holder 502. For another example, in an embodiment according to the invention, the destruction mechanism 528 includes a heating element, as discussed above, located against a bottom exterior surface of the drug holder 502 and thus be configured to heat the drug through the bottom exterior surface. For yet another example, in an embodiment according to the invention the destruction mechanism 528 includes a heating element, as discussed above, located against all side exterior surfaces of the drug holder 502 and thus be configured to heat the drug through all the side exterior surfaces. For still another example, the destruction mechanism 528 can include a rigid dowel configured to hit the drug holder 502, which is made of glass, such as a glass vial. The rigid dowel, e.g., a metal rod, etc., hitting the drug holder 502 is configured to break the drug holder 502, thereby causing the drug therein to leak onto a sponge or other absorbable material located adjacent to the drug holder 502 such that the sponge or other absorbable material absorbs the drug therein, similar to that discussed above. For another example, the drug holder 502 can be made from plastic, and the destruction mechanism 528 can include a heating element configured to heat the drug holder 502 and thereby melt or otherwise damage the drug holder 502 such that the drug leaks out of the drug holder 502 and onto a sponge or other absorbable material located adjacent to the drug holder 502 such that the sponge or other absorbable material absorbs the drug therein, similar to that discussed above. In this embodiment, the destruction mechanism 528 is configured to destroy the drug as well as the drug holders 502, which prevents the drug holder 502 from being reused.

As discussed above with respect to the drug delivery device 100 of FIG. 1, the drug delivery device 500 of FIG. 19 can include different features in different embodiments depending upon various requirements.

FIG. 20 illustrates one embodiment, according to the invention, of a drug delivery device 358 configured to expel a drug into a nose of a patient and configured to destroy drug on board the drug delivery device 358 using an exemplary embodiment of the destruction mechanism 528 that is located external to the drug delivery device's drug holder. The drug delivery device 358 is generally configured and used similar to the drug delivery device 500 of FIG. 19. The drug holder is located distal to the drug delivery device's cannula 360 that holds a piercing element configured to pierce or puncture a seal member of the drug holder, as discussed herein. The destruction mechanism 362 in this illustrated embodiment is located adjacent an opening 364 of the drug delivery device 358 just distal thereto in the drug delivery device 358.

A barrier 366 is located between the destruction mechanism 362 and the drug, as discussed above. In this illustrated embodiment, the barrier 366 is configured to selectively prevent the chemical 362 from entering an inner passageway 368 of the cannula 360 and thus from encountering the drug in the inner passageway 368 and/or from traveling within the inner passageway 368 to encounter the drug in the drug holder. FIG. 20 illustrates the barrier 366 in a first configuration preventing the chemical 362 from contacting the drug, either in the passageway 368 or in the drug holder. In response to occurrence of a predetermined event, as discussed above, the barrier 366 is configured to allow the chemical 362 to contact the drug. FIG. 21 illustrates the barrier 366 in a second configuration that allows the chemical 362 to prevent the drug from being delivered from the drug delivery device 358. In some embodiments, the chemical 362 can be sufficiently thick or otherwise configured to not travel distally in the inner passageway 368 in response to gravity. In such embodiments, as shown in FIG. 21, the chemical 362 is configured to block the opening 364 such that none of the drug can exit the drug delivery device 358 through the opening 364. In other embodiments, with the barrier 366 in the second configuration, the chemical 362 can be configured to enter and travel distally in the inner passageway 368 to encounter and destroy the drug in the passageway 368 and/or in the drug holder.

The barrier 366 can be configured to move from the first configuration to the second configuration in any of a variety of ways, as discussed above. For example, a processor can be configured to cause the barrier 366 to move from the first configuration to the second configuration by sliding distally relative to the cannula 360 in response to the occurrence of the predetermined trigger event.

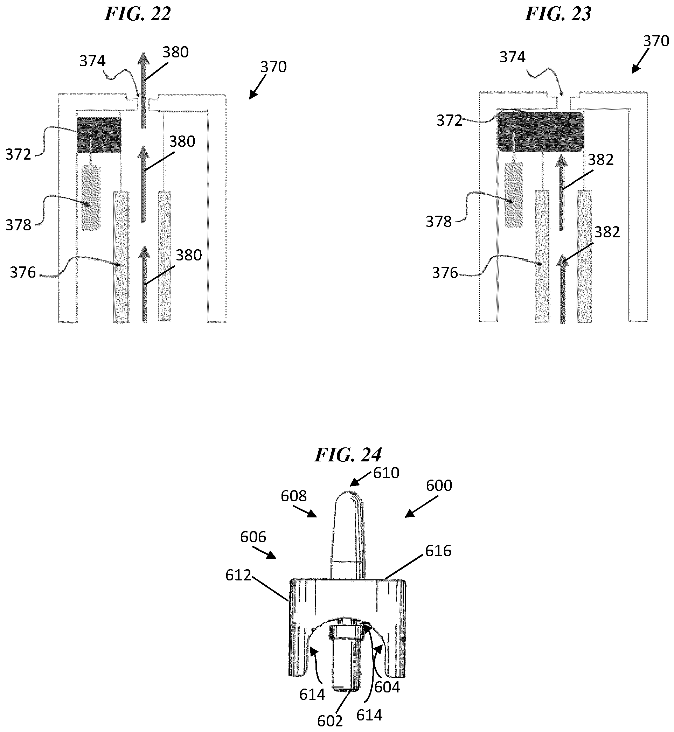

FIG. 22 illustrates another embodiment, according to the invention, of a drug delivery device 370 configured to expel a drug into a nose of a patient and configured to destroy drug on board the drug delivery device 370 using an exemplary embodiment of the destruction mechanism 528 that is located external to the drug delivery device's drug holder. The drug delivery device 370 is generally configured and used similar to the drug delivery device 358 of FIG. 20 except that the destruction mechanism 372 in this illustrated embodiment includes an inflatable bag, e.g., a balloon or bladder. Similar to the drug delivery device 358 of FIG. 20, in this illustrated embodiment the destruction mechanism 372 is located adjacent an opening 374 of the drug delivery device 370 just distal thereto in the drug delivery device 370. Also similar to the drug delivery device 358 of FIG. 20, in this illustrated embodiment the drug holder is located distal to the drug delivery device's cannula 376.

The destruction mechanism 372 is configured to move from a first configuration, shown in FIG. 22 to a second configuration, shown in FIG. 23. In the first configuration, the bag 372 is uninflated and does not obstruct the opening 374. The drug can thus exit through the opening 374 for delivery to a user from the drug delivery device 370 as shown by arrows 380 in FIG. 22. In the second configuration, the bag 372 is inflated and obstructs the opening 374. The drug thus cannot exit through the opening 374, as shown by arrows 382 in FIG. 23, and therefore cannot be delivered to a user from the drug delivery device 370.

The drug delivery device 370 includes an inflation mechanism 378 configured to cause the bag 372 to inflate and thereby move from the first configuration to the second configuration. The inflation mechanism 378 can have a variety of configurations. In this illustrated embodiment, the inflation mechanism 378 includes a gas canister configured to deliver air or other gas into the bag 372. With a valve in a closed position, the gas in the canister 378 cannot enter the bag 372. With the valve in an open position, the gas is allowed to exit the canister 378 and enter the bag 372 to inflate the bag 372 and thereby move the bag 372 from the first configuration to the second configuration. The valve can be actuated to move from the closed position to the open position in a variety of ways. For example, the valve can be an active valve, and a processor can be configured to cause the valve to move from the closed position to the open position in response to the occurrence of the predetermined trigger event. For another example, the valve can be a passive valve, and a processor can be configured to, in response to the occurrence of the predetermined trigger event, cause an activation mechanism of the canister 378 to push the gas in a direction toward the valve to cause the valve to automatically move from the closed position to the open position.

Regardless of whether a drug holder includes a destruction mechanism, e.g., as with the drug holder 102 and the destruction mechanism 128 of FIG. 1, or the drug holder is otherwise operatively coupled to the drug holder, e.g., as with the drug holder 502 and the destruction mechanism 528 of FIG. 19, a destruction indicator can be configured to indicate that the drug in the drug holder has been destroyed by the destruction mechanism. The destruction indicator can have a variety of configurations.

In an exemplary embodiment, the destruction indicator includes a dye pack configured to expel a dye in response to activation of the destruction mechanism. Visual observation of the dye indicates that the destruction mechanism has been activated and that, therefore, the drug has been destroyed. The dye can be configured to be visually observed with the naked eye, e.g., the dye is visible by visual observation, or with visual assistance, e.g., the dye is visible under black light or with other electronic assistance. The dye can include, e.g., red ink, black ink, food grade colorants, a substance visible under black light (e.g., nitric oxide, strontium aluminate, zinc sulfide, phospor, etc.), etc. The dye can be in liquid, powder, or tablet form. Examples of liquid dye include water tracing dye and natural dyes from minerals such as xanthene dyes and triphenlmethane dyes.

A processor can be configured to determine, based on data sensed by a sensor and communicated to the processor, whether the dye should be released from the dye pack. The sensed data can be any of the data discussed above, e.g., sensed geographic location to indicate an acceptable geographic location or an unacceptable geographic location, sensed temperature to indicate an acceptable temperature or an unacceptable temperature, sensed humidity to indicate an acceptable humidity or an unacceptable humidity, sensed pH to indicate an acceptable pH level or an unacceptable pH level, and/or sensed UV to indicate an acceptable UV level or an unacceptable UV level.

The dye pack can be located in a variety of locations. For example, the dye pack can be located on a box or packaging containing a single drug delivery device therein. The dye can thus be configured to stain or otherwise mark the box or packaging to indicate that the drug in the drug delivery device contained in the box or packaging has been destroyed. For another example, the dye pack can be located on a box or packaging containing a plurality of drug delivery devices therein. The dye can thus be configured to stain or otherwise mark the box or packaging to indicate that the drug in each of the drug delivery devices contained in the box or packaging has been destroyed.

In some embodiments, the dye pack can be used without a destruction mechanism being present. In such embodiments, the dye is configured to indicate that at least one anomalous event occurred, e.g., unacceptable geographic location, unacceptable temperature, unacceptable humidity, unacceptable pH level, unacceptable UV level, unacceptable DEA Registration Number, unacceptable identification code as indicated by RFID tag or other tag, with respect to the drug delivery device(s) associated with the expelled dye and that the associated drug delivery device(s) should therefore not be used for drug delivery.

FIG. 24 illustrates one embodiment of a drug delivery device 600 that can include a destruction mechanism, e.g., the destruction mechanism 128 of FIG. 1 or the destruction mechanism 528 of FIG. 19. The drug delivery device 600 is generally configured and used similar to that discussed above regarding FIGS. 1 and 19 and includes a drug holder 602, a piston 604, and a dispensing head 606 including a tip 608 and an opening 610. The dispensing head 606 has the piston 604 fixed thereto. From an inner end face of the piston 604 emanates an outlet channel, which is guided in valve-free manner to the opening 610 leading into the open and which is formed by an atomizing nozzle. The outlet channel becomes to be in fluid communication with a hollow interior of the drug holder 602 in response to the piston 604 puncturing or piercing a proximal seal member of the drug holder 602 as the drug holder 602 is pushed proximally, e.g., in a direction toward the opening 610. The dispensing head 606 includes a handle portion 612 configured to be handheld. Flattened sides of the handle portion 612 include two facing finger openings 614 configured to receive a thumb of a user's hand with the index and middle finger of this hand on either side of a discharge connection of the handle portion 612 on an outside of the handle portion's end wall 616 or elsewhere on the handle portion 612. By moving together the thumb and the two other fingers the drug holder 602 can be displaced with respect to the piston 604 or the dispensing head 606 and the drug consequently sprayed out of the drug holder 602 through a discharge channel of the drug delivery device 600 and through the opening 610. The drug delivery device 600 is further described in U.S. Pat. No. 4,946,069 entitled "Dispenser For Manually Discharging Flowable Media" issued Aug. 7, 1990.

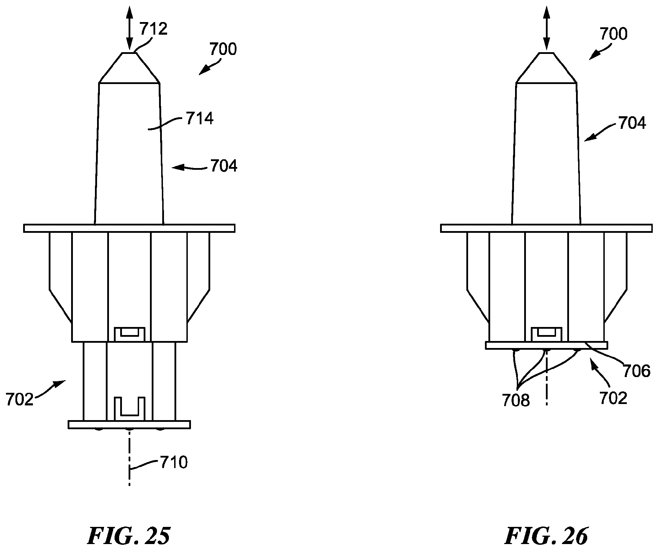

FIGS. 25 and 26 illustrate another embodiment of a drug delivery device 700 that can include a destruction mechanism, e.g., the destruction mechanism 128 of FIG. 1 or the destruction mechanism 528 of FIG. 19. The drug delivery device 700 is generally configured and used similar to that discussed above regarding FIGS. 1 and 19. FIG. 25 shows this illustrated embodiment of a drug delivery device 700 in an initial or pre-use configuration, and FIG. 26 shows the drug delivery device 700 in an actuated or post use configuration. In the post use configuration, a body 702 of the drug delivery device 700 has been inserted into a dispensing head 704 of the drug delivery device 700 such that only an endplate 706 of the body 702 is visible.

The body 702, which includes a drug holder therein, is configured to be inserted into the dispensing head 704 substantially along a first axis 710. A person skilled in the art will appreciate that the body 702 may not be inserted precisely along the first axis 710 but nevertheless be considered to be inserted substantially along the first axis 710 due to any number of factors, such as manufacturing tolerances and sensitivity of measurement equipment. The body 702 defines an actuator of the drug delivery device 700 that is configured to be manually moved by a user to be inserted into the dispensing head 704 and cause drug delivery. The endplate 706 includes a gripping feature 708 configured to facilitate actuation by increasing grip of the body's surface for a finger of a user. The gripping feature 708 includes surface projections in this illustrated embodiment but can have other configurations, such as a textured surface, a finger depression, etc.

During use of the drug delivery device 700, an insertion force is applied to the body 702 such that the body 702 slides proximally, e.g., toward an opening 712 in a tip 714 of the dispensing head 704, relative to the dispensing head 704. As the body 702 is moved in the proximal direction, a piercing element punctures or pierces a proximal seal member of the drug holder. Further movement of the body 702 in the proximal direction causes a piercer stop of a holder holding the piercing element in the dispensing head 704 to come into contact with the drug holder. The contact between the piercer stop and the drug holder are configured to prevent further insertion of the body 702 in the proximal direction, which can act as an indicator to a user of the drug delivery device 700 that actuation has been completed.

The drug delivery device 700 is further described in U.S. Pat. Pub. No. 2018/0361085 entitled "Nasal Spray Assembly" published Dec. 20, 2018.

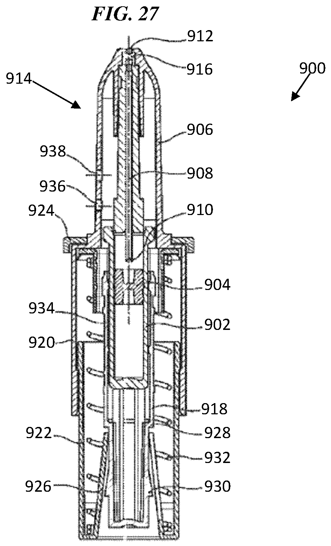

FIG. 27 illustrates one embodiment of a drug delivery device 900 that can include a destruction mechanism, e.g., the destruction mechanism 128 of FIG. 1 or the destruction mechanism 528 of FIG. 19. The drug delivery device 900 is generally configured and used similar to that discussed above regarding FIGS. 1 and 19 and includes a drug holder 902 containing two doses of drug therein. A dispensing mechanism, such as a piston 904, is mounted to slide in the drug holder 902. In a pre-actuation position of the drug delivery device 900, shown in FIG. 27, the piston 904 acts as a stopper, isolating the drug. The drug delivery device 900 also includes a dispensing head 906 that is assembled on the drug holder 902 and that is configured to be axially movable relative to the drug holder 902. In particular, an axial movement of the dispensing head 906 relative to the drug holder 902 causes the piston 904 to move in the drug holder 902, and thus causes the drug contained in the drug holder 902 to be dispensed. The dispensing head 906 includes a dispenser channel 908 that extends from a perforator tip 910 to an opening 912 in a tip 914 of the dispensing head 906. Proximal to or upstream of the opening 912, a spray profile 916 can be provided that is configured to dispense the drug in the form of spray.

The drug holder 902 is fastened in a body 918 that is thus secured to the drug holder 902 and that moves together with the drug holder 902.

The dispensing head 906 includes a bottom side skirt 920 that is adapted to cooperate with an actuator 922 of the drug delivery device 900. A finger-rest element 924 can be assembled around the dispensing head 906 or can be formed integrally therewith.

The actuator 922 is configured to be axially movable inside the side skirt 920 of the dispensing head 906 so as to perform successive actuations of the drug delivery device 900. The actuator 922 includes at least one sloping tab 926 that is configured to cooperate with projections 928, 930 of the body 918 so as to perform successive actuations. A return spring 932 is mounted between the actuator 922 and the dispensing head 906 so as to return the actuator 922 into its start position after each actuation.

The drug delivery device 900 includes a device indicator 934 configured to indicate to a user whether a first dose has been dispensed and whether a second dose has been dispensed. In this way, the user knows exactly what the situation is, and whether or not the first dose has been dispensed. The indicator 934 is configured to cooperate with viewing windows 936, 938 that are formed in the dispensing head 906. The viewing windows 936, 938 are formed in a side wall of the dispensing head 906, clearly visible to the user when the drug delivery device 900 is held in the hand.

The drug delivery device 900 is further described in U.S. Pat. No. 9,555,950 entitled "Fluid Product Dispensing Device" issued Jan. 31, 2017.

As discussed herein, one or more aspects or features of the subject matter described herein, e.g., electronic components such as a processor, memory, communications interface, etc., can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computer system may include clients and servers. A client and server are generally remote from each other and typically interact through a communications network, e.g., the Internet, a wireless wide area network, a local area network, a wide area network, or a wired network. The relationship of client and server arises by virtue of computer programs running on the respective computers and having a client-server relationship to each other.

The computer programs, which can also be referred to as programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein, for example a user interface, can be implemented on a computer having a display screen, such as for example a cathode ray tube (CRT) or a liquid crystal display (LCD) or a light emitting diode (LED) monitor for displaying information to the user. The display screen can allow input thereto directly (e.g., as a touch screen) or indirectly (e.g., via an input device such as a keypad or voice recognition hardware and software). Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input.

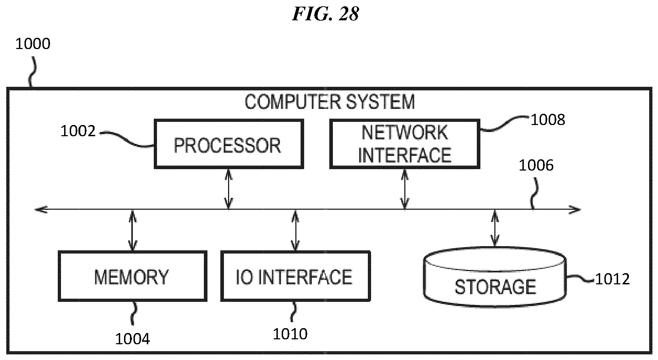

FIG. 28 illustrates one exemplary embodiment of a computer system 1000. The computer system 1000 includes a processor 1002 configured to control the operation of the computer system 1000. The processor 1002 can include any type of microprocessor or central processing unit (CPU), including programmable general-purpose or special-purpose microprocessors and/or any one of a variety of proprietary or commercially available single or multi-processor systems. The computer system 1000 also includes a memory 1004 configured to provide temporary storage for code to be executed by the processor 1002 or for data acquired from one or more users, storage devices, sensors, and/or databases. The memory 1004 can include read-only memory (ROM), flash memory, one or more varieties of random access memory (RAM) (e.g., static RAM (SRAM), dynamic RAM (DRAM), or synchronous DRAM (SDRAM)), and/or a combination of memory technologies.

The various elements of the computer system 1000 are coupled to a bus system 1006. The illustrated bus system 1006 is an abstraction that represents any one or more separate physical busses, communication lines/interfaces, and/or multi-drop or point-to-point connections, connected by appropriate bridges, adapters, and/or controllers. The computer system 1000 also includes a network interface 1008 (also referred to herein as a communications interface), an input/output (IO) interface(s) 1010, and a storage device 1012.

The communications interface 1008 is configured to enable the computer system to communicate with remote devices, e.g., other computer systems, and can be, for example, remote desktop connection interfaces, Ethernet adapters, and/or other local area network (LAN) adapters. The IO interface 1010 includes one or more interface components to connect the computer system 1000 with other electronic equipment. For example, the IO interface 1010 can include high speed data ports, such as universal serial bus (USB) ports, 13104 ports, Wi-Fi, Bluetooth, etc. Additionally, the computer system 1000 can be accessible to a human user, and thus the IO interface 1010 can include displays, speakers, keyboards, pointing devices, and/or various other video, audio, or alphanumeric interfaces. The storage device 1012 includes any conventional medium for storing data in a non-volatile and/or non-transient manner. The storage device 1012 is thus configured to hold data and/or instructions in a persistent state in which the value(s) are retained despite interruption of power to the computer system. The storage device 1012 can include one or more hard disk drives, flash drives, USB drives, optical drives, various media cards, diskettes, compact discs, and/or any combination thereof and can be directly connected to the computer system or remotely connected thereto, such as over a network. In an exemplary embodiment, the storage device 1012 includes a tangible or non-transitory computer readable medium configured to store data, e.g., a hard disk drive, a flash drive, a USB drive, an optical drive, a media card, a diskette, or a compact disc.

The elements illustrated in FIG. 28 can be some or all of the elements of a single physical machine. In addition, not all of the illustrated elements need to be located on or in the same physical machine.

The computer system 1000 can include a web browser for retrieving web pages or other markup language streams, presenting those pages and/or streams (visually, aurally, or otherwise), executing scripts, controls and other code on those pages/streams, accepting user input with respect to those pages/streams (e.g., for purposes of completing input fields), issuing HyperText Transfer Protocol (HTTP) requests with respect to those pages/streams or otherwise (e.g., for submitting to a server information from the completed input fields), and so forth. The web pages or other markup language can be in HyperText Markup Language (HTML) or other conventional forms, including embedded Extensible Markup Language (XML), scripts, controls, and so forth. The computer system 1000 can also include a web server for generating and/or delivering the web pages to client computer systems.

The computer system 1000 can also include any of a variety of other software and/or hardware components, including by way of example, operating systems and database management systems. Although an exemplary computer system is depicted and described herein, it will be appreciated that this is for sake of generality and convenience. In other embodiments, the computer system 1000 may differ in architecture and operation from that shown and described here. For example, the memory 1004 and storage device 1012 can be integrated together or the communications interface 1008 can be omitted if communication with another computer system is not necessary.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. **In** particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure.

## Claims

1. A drug delivery system, comprising:
a tip (112, 512, 608, 714, 914) configured to be positioned in a nose of a patient, the tip having an opening (108, 508, 364, 374, 610, 712, 912) therein;
a drug holder (102, 200, 300, 300A, 312, 330, 302B, 502, 602) containing a drug therein that is configured to exit through the opening; and
a destruction mechanism (128, 306, 306A, 314, 332, 342, 300B, 528, 362, 372) configured to be activated and thereby destroy substantially all of the drug in the drug holder; and wherein one of the following options is fulfilled:
a) the destruction mechanism (128, 306, 306A, 314, 332, 342, 300B) is disposed in the drug holder, wherein the destruction mechanism (128, 306, 306A, 314, 332, 528, 362) includes a chemical or an absorbable material configured to absorb the drug therein, wherein the system further comprises a barrier (310, 324, 338, 366) configured to prevent the drug and the chemical or an absorbable material from coming into contact with one another, wherein the activation of the destruction mechanism causes a piercing element to break the barrier to allow the drug and the chemical or the absorbable material to come into contact with one another;
b) the system further comprises a body with the drug holder (502) disposed therein; wherein the destruction mechanism (528) is disposed in the body external to the drug holder and wherein the destruction mechanism includes a heating element configured to operatively engage the drug in the drug holder to destroy the drug;
c) the system further comprises a body with the drug holder (502) disposed therein; wherein the destruction mechanism (362, 372) is disposed in the body external to the drug holder (502) and wherein the destruction mechanism is adjacent the opening (364, 374) of the drug delivery system just distal thereto in the drug delivery system.

2. The drug delivery system of claim 1, wherein the destruction mechanism is configured to destroy substantially all of the drug in the drug holder by causing the drug to lose its effectiveness as a treatment or by making the drug no longer deliverable from the drug delivery system as a nasal spray.

3. The system of claim 1a), wherein the destruction mechanism (128, 306, 306A, 314, 332, 528, 362) includes the chemical; optionally wherein the chemical includes one of silica, bleach, charcoal, glycerol, sodium polyacrylate, activated carbon, zeolytes, and acid;
or the system of claim 1c), wherein the destruction mechanism includes a chemical; optionally wherein one or both of the following is fulfilled:
a) the system further comprises a barrier configured to prevent the drug and the chemical from coming into contact with one another, wherein the activation of the destruction mechanism causes the barrier to break to allow the drug and the chemical to come into contact with one another;
b) the chemical includes one of silica, bleach, charcoal, glycerol, sodium polyacrylate, activated carbon, zeolytes, and acid.

4. The system of claim 1a), wherein the destruction mechanism (332) is configured to destroy substantially all of the drug in the drug holder by making the drug no longer deliverable from the drug delivery system as a nasal spray, and the destruction mechanism includes the absorbable material (332) configured to absorb the drug therein; optionally wherein the absorbable material (332) includes silica or a sponge; or
the system of claim 1c), wherein the destruction mechanism (332) is configured to destroy substantially all of the drug in the drug holder by making the drug no longer deliverable from the drug delivery system as a nasal spray, and the destruction mechanism includes an absorbable material (332) configured to absorb the drug therein; optionally wherein the absorbable material (332) includes silica or a sponge.

5. The system of any one of the preceding claims, further comprising an actuator (106, 506, 702, 922) configured to be actuated by a user to cause the drug to be delivered out of the opening; optionally wherein after the activation of the destruction mechanism, the actuation of the actuator (106, 506, 702, 922) cannot cause the drug to be delivered out of the opening.

6. The system of any one of the preceding claims, further comprising a processor (122, 522, 1002) configured to cause the activation of the destruction mechanism in response to occurrence of a predetermined trigger event, optionally wherein the system further comprises a body with the drug holder disposed therein;
wherein the processor (122, 522, 1002) is disposed in the body.

7. The system of claim 6, further comprising a communications interface (120, 520, 1008) configured to electronically receive an instruction from an external device;
wherein the external device includes the processor (122, 522, 1002).

8. The system of claim 6 or 7, wherein one of the following options is fulfilled:
a) the system further comprises a location sensor (118, 516) configured to monitor geographic location;
wherein the predetermined trigger event includes the geographic location sensed by the location sensor being an unacceptable geographic location as determined by the processor (122, 522, 1002);
b) the system further comprises a temperature sensor (118, 516) configured to monitor temperature;
wherein the predetermined trigger event includes the temperature sensed by the temperature sensor being an unacceptable temperature as determined by the processor (122, 522, 1002);
c) the system further comprises a humidity sensor (118, 516) configured to monitor humidity;
wherein the predetermined trigger event includes the humidity sensed by the humidity sensor being an unacceptable humidity as determined by the processor (122, 522, 1002);
d) the system further comprises a pH sensor (118, 516) configured to monitor pH;
wherein the predetermined trigger event includes the pH sensed by the pH sensor being an unacceptable pH as determined by the processor (122, 522, 1002)
e) the system further comprises an ultraviolet (UV) sensor (118, 516) configured to monitor UV exposure;
wherein the predetermined trigger event includes the UV exposure sensed by the UV sensor being an unacceptable UV exposure as determined by the processor (122, 522, 1002).

9. The system of any one of claims 6 to 8, wherein the predetermined trigger event includes the processor (122, 522, 1002) identifying an unacceptable Drug Enforcement Administration (DEA) Registration Number.

10. The system of any one of claims 6 to 9, further comprising a tag storing data therein;
wherein the predetermined trigger event includes the processor (122, 522, 1002) identifying unacceptable data received from the tag.

11. The system of any one of claims 1 to 5, wherein the destruction mechanism (128, 306, 306A, 314, 332, 342, 300B, 528, 362, 372) is configured to be automatically activated in response to occurrence of a predetermined trigger event.

12. The system of claim 11, wherein one or more of the following options is fulfilled:
a) the system further comprises a location sensor (118, 516) configured to monitor geographic location;
wherein the predetermined trigger event includes the geographic location sensed by the location sensor (118, 516) being an unacceptable geographic location;
b) the system further comprises a temperature sensor (118, 516) configured to monitor temperature;
wherein the predetermined trigger event includes the temperature sensed by the temperature sensor (118, 516) being an unacceptable temperature;
c) the system further comprises a humidity sensor (118, 516) configured to monitor humidity;
wherein the predetermined trigger event includes the humidity sensed by the humidity sensor (118, 516) being an unacceptable humidity;
d) the system further comprises a pH sensor (118, 516) configured to monitor pH;
wherein the predetermined trigger event includes the pH sensed by the pH sensor being an unacceptable pH;
e) the system further comprises an ultraviolet (UV) sensor configured to monitor UV exposure;
wherein the predetermined trigger event includes the UV exposure sensed by the UV sensor being an unacceptable UV exposure.

13. The system of claim 11 or 12, wherein the predetermined trigger event includes identification of an unacceptable Drug Enforcement Administration (DEA) Registration Number.

14. The system of claim any one of claims 11 to 13, further comprising a tag storing data therein;
wherein the predetermined trigger event includes identification of unacceptable data received from the tag.

15. The system of any one of the preceding claims, wherein the drug is one of ketamine, esketamine, naloxone, and sumatriptan.

## Patentansprüche

1. Arzneimittelabgabesystem, umfassend:
eine Spitze (112, 512, 608, 714, 914), die konfiguriert ist, um in einer Nase eines Patienten positioniert zu werden, wobei die Spitze eine Öffnung (108, 508, 364, 374, 610, 712, 912) darin aufweist;
einen Arzneimittelhalter (102, 200, 300, 300A, 312, 330, 302B, 502, 602), der ein Arzneimittel darin enthält, das konfiguriert ist, um durch die Öffnung auszutreten; und
einen Zerstörungsmechanismus (128, 306, 306A, 314, 332, 342, 300B, 528, 362, 372), der konfiguriert ist, um aktiviert zu werden und dadurch im Wesentlichen das gesamte Arzneimittel in dem Arzneimittelhalter zu zerstören; wobei optional eine der folgenden Optionen erfüllt ist:
a) der Zerstörungsmechanismus (128, 306, 306A, 314, 332, 342, 300B) ist in dem Arzneimittelhalter angeordnet, wobei der Zerstörungsmechanismus (128, 306, 306A, 314, 332, 528, 362) eine Chemikalie oder ein absorbierbares Material einschließt, das konfiguriert ist, um das Arzneimittel darin zu absorbieren, wobei das System ferner eine Barriere (310, 324, 338, 366) umfasst, die konfiguriert ist, um zu verhindern, dass das Arzneimittel und die Chemikalie oder das absorbierbare Material miteinander in Kontakt kommen, wobei die Aktivierung des Zerstörungsmechanismus bewirkt, dass ein Durchstoßelement die Barriere durchbricht, um zu ermöglichen, dass das Arzneimittel und die Chemikalie oder das absorbierbare Material miteinander in Kontakt kommen;
b) das System umfasst ferner einen Körper mit dem darin angeordneten Arzneimittelhalter (502); wobei der Zerstörungsmechanismus (528) in dem Körper außerhalb des Arzneimittelhalters angeordnet ist und wobei der Zerstörungsmechanismus ein Heizelement einschließt, das konfiguriert ist, um funktionsmäßig mit dem Arzneimittel in dem Arzneimittelhalter in Eingriff zu treten, um das Arzneimittel zu zerstören;
c) das System umfasst ferner einen Körper mit dem darin angeordneten Arzneimittelhalter (502); wobei der Zerstörungsmechanismus (362, 372) in dem Körper außerhalb des Arzneimittelhalters (502) angeordnet ist und wobei der Zerstörungsmechanismus der Öffnung (364, 374) des Arzneimittelabgabesystems unmittelbar distal davon in dem Arzneimittelabgabesystem benachbart ist.

2. Arzneimittelabgabesystem nach Anspruch 1, wobei der Zerstörungsmechanismus konfiguriert ist, um im Wesentlichen das gesamte Arzneimittel in dem Arzneimittelhalter zu zerstören, indem bewirkt wird, dass das Arzneimittel seine Wirksamkeit als Behandlung verliert oder indem das Arzneimittel nicht länger aus dem Arzneimittelabgabesystem als Nasenspray abgebbar gemacht wird.

3. System nach Anspruch 1a), wobei der Zerstörungsmechanismus (128, 306, 306A, 314, 332, 528, 362) die Chemikalie einschließt; wobei optional die Chemikalie eines aus Siliciumdioxid, Bleichmittel, Holzkohle, Glycerol, Natriumpolyacrylat, Aktivkohle, Zeolithen und Säure einschließt;
oder System nach Anspruch 1c), wobei der Zerstörungsmechanismus eine Chemikalie einschließt; wobei optional eine oder beide der folgenden Optionen erfüllt sind:
a) das System umfasst ferner eine Barriere, die konfiguriert ist, um zu verhindern, dass das Arzneimittel und die Chemikalie miteinander in Kontakt kommen, wobei die Aktivierung des Zerstörungsmechanismus bewirkt, dass die Barriere bricht, um zu ermöglichen, dass das Arzneimittel und die Chemikalie miteinander in Kontakt kommen;
b) die Chemikalie schließt eines aus Siliciumdioxid, Bleichmittel, Holzkohle, Glycerol, Natriumpolyacrylat, Aktivkohle, Zeolithen und Säure ein.

4. System nach Anspruch 1a), wobei der Zerstörungsmechanismus (332) konfiguriert ist, um im Wesentlichen das gesamte Arzneimittel in dem Arzneimittelhalter zu zerstören, indem das Arzneimittel nicht mehr aus dem Arzneimittelabgabesystem als Nasenspray abgebbar gemacht wird, und der Zerstörungsmechanismus das absorbierbare Material (332) einschließt, das konfiguriert ist, um das Arzneimittel darin zu absorbieren; wobei optional das absorbierbare Material (332) Siliciumdioxid oder einen Schwamm einschließt; oder
System nach Anspruch 1c), wobei der Zerstörungsmechanismus (332) konfiguriert ist, um im Wesentlichen das gesamte Arzneimittel in dem Arzneimittelhalter zu zerstören, indem das Arzneimittel nicht mehr aus dem Arzneimittelabgabesystem als Nasenspray abgebbar gemacht wird, und der Zerstörungsmechanismus ein absorbierbares Material (332) einschließt, das konfiguriert ist, um das Arzneimittel darin zu absorbieren; wobei optional das absorbierbare Material (332) Siliciumdioxid oder einen Schwamm einschließt.

5. System nach einem der vorstehenden Ansprüche, ferner umfassend einen Aktuator (106, 506, 702, 922), der konfiguriert ist, um durch einen Benutzer betätigt zu werden, um zu bewirken, dass das Arzneimittel aus der Öffnung abgegeben wird; wobei optional nach der Aktivierung des Zerstörungsmechanismus die Betätigung des Aktuators (106, 506, 702, 922) nicht bewirken kann, dass das Arzneimittel aus der Öffnung abgegeben wird.

6. System nach einem der vorstehenden Ansprüche, ferner umfassend einen Prozessor (122, 522, 1002), der konfiguriert ist, um die Aktivierung des Zerstörungsmechanismus als Reaktion auf das Auftreten eines vorgegebenen Auslöseereignisses zu veranlassen, wobei optional das System ferner einen Körper mit dem darin angeordneten Arzneimittelhalter umfasst;
wobei der Prozessor (122, 522, 1002) in dem Körper angeordnet ist.

7. System nach Anspruch 6, ferner umfassend eine Kommunikationsschnittstelle (120, 520, 1008), die konfiguriert ist, um eine Anweisung von einer externen Vorrichtung elektronisch zu empfangen;
wobei die externe Vorrichtung den Prozessor (122, 522, 1002) einschließt.

8. Verfahren nach Anspruch 6 oder 7, wobei eine der folgenden Optionen erfüllt ist:
a) das System umfasst ferner einen Standortsensor (118, 516), der konfiguriert ist, um den geografischen Standort zu überwachen;
wobei das vorgegebene Auslöseereignis einschließt, dass der durch den Standortsensor erfasste geografische Standort ein geografischer Standort ist, der durch den Prozessor (122, 522, 1002) als unzulässig ermittelt wurde;
b) das System umfasst ferner einen Temperaturfühler (118, 516), der konfiguriert ist, um die Temperatur zu überwachen;
wobei das vorgegebene Auslöseereignis einschließt, dass durch den Temperatursensor erfasste Temperatur eine Temperatur ist, die durch den Prozessor (122, 522, 1002) als unzulässig ermittelt wurde;
c) das System umfasst ferner einen Feuchtesensor (118, 516), der konfiguriert ist, um die Feuchte zu überwachen;
wobei das vorgegebene Auslöseereignis einschließt, dass die durch den Feuchtesensor erfasste Feuchte eine Feuchte ist, die durch den Prozessor (122, 522, 1002) als unzulässig ermittelt wurde;
d) das System umfasst ferner einen pH-Wert-Sensor (118, 516), der konfiguriert ist, um den pH-Wert zu überwachen;
wobei das vorgegebene Auslöseereignis einschließt, dass der durch den pH-Wert-Sensor erfasste pH-Wert ein pH-Wert ist, der durch den Prozessor (122, 522, 1002) als unzulässig ermittelt wurde
e) das System umfasst ferner einen Ultraviolettsensor (UV-Sensor) (118, 516), der konfiguriert ist, um die UV-Exposition zu überwachen;
wobei das vorgegebene Auslöseereignis einschließt, dass die durch den UV-Sensor erfasste UV-Exposition eine UV-Exposition ist, die durch den Prozessor (122, 522, 1002) als unzulässig ermittelt wurde.

9. System nach einem der Ansprüche 6 bis 8, wobei das vorgegebene Auslöseereignis einschließt, dass der Prozessor (122, 522, 1002) eine unzulässige Registrierungsnummer der Drug Enforcement Administration (DEA-Registrierungsnummer) erkennt.

10. Verfahren nach einem der Ansprüche 6 bis 9, ferner umfassend ein Tag, das Daten darin speichert;
wobei das vorgegebene Auslöseereignis einschließt, dass der Prozessor (122, 522, 1002) unzulässige Daten erkennt, die von dem Tag empfangen wurden.

11. System nach einem der Ansprüche 1 bis 5, wobei der Zerstörungsmechanismus (128, 306, 306A, 314, 332, 342, 300B, 528, 362, 372) konfiguriert ist, um automatisch als Reaktion auf das Auftreten eines vorgegebenen Auslöseereignisses aktiviert zu werden.

12. Verfahren nach Anspruch 11, wobei eine oder mehrere der folgenden Optionen erfüllt sind:
a) das System umfasst ferner einen Standortsensor (118, 516), der konfiguriert ist, um den geografischen Standort zu überwachen;
wobei das vorgegebene Auslöseereignis einschließt, dass der durch den Standortsensor (118, 516) erfasste geografische Standort ein unzulässiger geografischer Standort ist;
b) das System umfasst ferner einen Temperaturfühler (118, 516), der konfiguriert ist, um die Temperatur zu überwachen;
wobei das vorgegebene Auslöseereignis einschließt, dass die durch den Temperatursensor (118, 516) erfasste Temperatur eine unzulässige Temperatur ist;
c) das System umfasst ferner einen Feuchtesensor (118, 516), der konfiguriert ist, um die Feuchte zu überwachen;
wobei das vorgegebene Auslöseereignis einschließt, dass die durch den Feuchtesensor (118, 516) erfasste Feuchte eine unzulässige Feuchte ist;
d) das System umfasst ferner einen pH-Wert-Sensor (118, 516), der konfiguriert ist, um den pH-Wert zu überwachen;
wobei das vorgegebene Auslöseereignis einschließt, dass der durch den pH-Wert-Sensor erfasste pH-Wert ein unzulässiger pH-Wert ist;
e) das System umfasst ferner einen Ultraviolettsensor (UV-Sensor), der konfiguriert ist, um die UV-Exposition zu überwachen;
wobei das vorgegebene Auslöseereignis einschließt, dass die durch den UV-Sensor erfasste UV-Exposition eine unzulässige UV-Exposition ist.

13. System nach Anspruch 11 oder 12, wobei das vorgegebene Auslöseereignis die Erkennung einer unzulässigen Registrierungsnummer der Drug Enforcement Administration (DEA-Registrierungsnummer) einschließt.

14. Verfahren nach einem der Ansprüche 11 bis 13, ferner umfassend ein Tag, das Daten darin speichert;
wobei das vorgegebene Auslöseereignis die Erkennung von unzulässigen Daten einschließt, die von dem Tag empfangen wurden.

15. System nach einem der vorstehenden Ansprüche, wobei das Arzneimittel eines aus Ketamin, Esketamin, Naloxon oder Sumatriptan ist.

## Revendications

1. Système d'administration de médicament, comprenant :
un embout (112, 512, 608, 714, 914) conçu pour être positionné dans un nez d'un patient, l'embout ayant une ouverture (108, 508, 364, 374, 610, 712, 912) dans celui-ci ;
un stockage de médicament (102, 200, 300, 300A, 312, 330, 302B, 502, 602) contenant un médicament dans celui-ci qui est conçu pour sortir par l'ouverture ; et
un mécanisme de destruction (128, 306, 306A, 314, 332, 342, 300B, 528, 362, 372) conçu pour être activé et détruire de ce fait sensiblement la totalité du médicament dans le stockage de médicament ; et dans lequel l'une des options suivantes est respectée :
a) le mécanisme de destruction (128, 306, 306A, 314, 332, 342, 300B) est disposé dans le stockage de médicament, dans lequel le mécanisme de destruction (128, 306, 306A, 314, 332, 528, 362) comporte une substance chimique ou un matériau absorbable conçu pour absorber le médicament dans celui-ci, dans lequel le système comprend en outre une barrière (310, 324, 338, 366) conçue pour empêcher le médicament et la substance chimique ou un matériau absorbable de venir en contact l'un avec l'autre, dans lequel l'activation du mécanisme de destruction amène un élément de perçage à rompre la barrière pour permettre au médicament et à la substance chimique ou au matériau absorbable de venir en contact l'un avec l'autre ;
b) le système comprend en outre un corps avec le stockage de médicament (502) disposé dans celui-ci ; dans lequel le mécanisme de destruction (528) est disposé dans le corps à l'extérieur du stockage de médicament et dans lequel le mécanisme de destruction comporte un élément chauffant conçu pour venir fonctionnellement en prise avec le médicament dans le stockage de médicament pour détruire le médicament ;
c) le système comprend en outre un corps avec le stockage de médicament (502) disposé dans celui-ci ; dans lequel le mécanisme de destruction (362, 372) est disposé dans le corps à l'extérieur du stockage de médicament (502) et dans lequel le mécanisme de destruction est adjacent à l'ouverture (364, 374) du système d'administration de médicament juste distale par rapport à celui-ci dans le système d'administration de médicament.

2. Système d'administration de médicament selon la revendication 1, dans lequel le mécanisme de destruction est conçu pour détruire sensiblement la totalité du médicament dans le stockage de médicament en amenant le médicament à perdre son efficacité en tant que traitement ou en faisant en sorte que le médicament n'est plus administrable par le système d'administration de médicament en tant que pulvérisation nasale.

3. Système selon la revendication 1a), dans lequel le mécanisme de destruction (128, 306, 306A, 314, 332, 528, 362) comporte la substance chimique ; facultativement dans lequel la substance chimique comporte l'un parmi silice, eau de Javel, charbon de bois, glycérol, polyacrylate de sodium, charbon actif, zéolites et acide ;
ou système selon la revendication 1c), dans lequel le mécanisme de destruction comporte une substance chimique ; facultativement dans lequel l'un et/ou l'autre de ce qui suit sont respectés :
a) le système comprend en outre une barrière conçue pour empêcher le médicament et la substance chimique de venir en contact l'un avec l'autre, dans lequel l'activation du mécanisme de destruction amène la barrière à se rompre pour permettre au médicament et à la substance chimique de venir en contact l'un avec l'autre ;
b) la substance chimique comporte l'un parmi silice, eau de Javel, charbon de bois, glycérol, polyacrylate de sodium, charbon actif, zéolites et acide.

4. Système selon la revendication 1a), dans lequel le mécanisme de destruction (332) est conçu pour détruire sensiblement la totalité du médicament dans le stockage de médicament en faisant en sorte que le médicament ne soit plus administrable par le système d'administration de médicament en tant que pulvérisation nasale, et le mécanisme de destruction comporte le matériau absorbable (332) conçu pour absorber le médicament dans celui-ci ; facultativement dans lequel le matériau absorbable (332) comporte de la silice ou une éponge ; ou
système selon la revendication 1c), dans lequel le mécanisme de destruction (332) est conçu pour détruire sensiblement la totalité du médicament dans le stockage de médicament en faisant en sorte que le médicament ne soit plus administrable par le système d'administration de médicament en tant que pulvérisation nasale, et le mécanisme de destruction comporte un matériau absorbable (332) conçu pour absorber le médicament dans celui-ci ; facultativement dans lequel le matériau absorbable (332) comporte de la silice ou une éponge.

5. Système selon l'une quelconque des revendications précédentes, comprenant en outre un actionneur (106, 506, 702, 922) conçu pour être actionné par un utilisateur pour amener le médicament à être administré par l'ouverture ; facultativement dans lequel après l'activation du mécanisme de destruction, l'actionnement de l'actionneur (106, 506, 702, 922) ne peut pas amener le médicament à être administré par l'ouverture.

6. Système selon l'une quelconque des revendications précédentes, comprenant en outre un processeur (122, 522, 1002) configuré pour amener l'activation du mécanisme de destruction en réponse à l'occurrence d'un événement déclencheur prédéterminé, facultativement dans lequel le système comprend en outre un corps avec le stockage de médicament disposé dans celui-ci ;
dans lequel le processeur (122, 522, 1002) est disposé dans le corps.

7. Système selon la revendication 6, comprenant en outre une interface de communications (120, 520, 1008) configurée pour recevoir électroniquement une instruction en provenance d'un dispositif externe ;
dans lequel le dispositif externe comporte le processeur (122, 522, 1002).

8. Système selon la revendication 6 ou 7, dans lequel l'une des options suivantes est respectée :
a) le système comprend en outre un capteur de localisation (118, 516) configuré pour surveiller une localisation géographique ;
dans lequel l'événement déclencheur prédéterminé comporte la localisation géographique captée par le capteur de localisation étant une localisation géographique inacceptable telle que déterminée par le processeur (122, 522, 1002) ;
b) le système comprend en outre un capteur de température (118, 516) configuré pour surveiller la température ;
dans lequel l'événement déclencheur prédéterminé comporte la température captée par le capteur de température étant une température inacceptable telle que déterminée par le processeur (122, 522, 1002) ;
c) le système comprend en outre un capteur d'humidité (118, 516) configuré pour surveiller l'humidité ;
dans lequel l'événement déclencheur prédéterminé comporte l'humidité captée par le capteur d'humidité étant une humidité inacceptable telle que déterminée par le processeur (122, 522, 1002) ;
d) le système comprend en outre un capteur de pH (118, 516) configuré pour surveiller le pH ;
dans lequel l'événement déclencheur prédéterminé comporte le pH capté par le capteur de pH étant un pH inacceptable tel que déterminé par le processeur (122, 522, 1002)
e) le système comprend en outre un capteur d'ultraviolets (UV) (118, 516) configuré pour surveiller une exposition aux UV ;
dans lequel l'événement déclencheur prédéterminé comporte l'exposition aux UV captée par le capteur UV étant une exposition aux UV inacceptable telle que déterminée par le processeur (122, 522, 1002).

9. Système selon l'une quelconque des revendications 6 à 8, dans lequel l'événement déclencheur prédéterminé comporte le processeur (122, 522, 1002) identifiant un numéro d'enregistrement Drug Enforcement Administration (DEA) inacceptable.

10. Système selon l'une quelconque des revendications 6 à 9, comprenant en outre une étiquette de stockage de données dans celle-ci ;
dans lequel l'événement déclencheur prédéterminé comporte le processeur (122, 522, 1002) identifiant des données inacceptables reçues de l'étiquette.

11. Système selon l'une quelconque des revendications 1 à 5, dans lequel le mécanisme de destruction (128, 306, 306A, 314, 332, 342, 300B, 528, 362, 372) est configuré pour être automatiquement activé en réponse à l'occurrence d'un événement déclencheur prédéterminé.

12. Système selon la revendication 11, dans lequel une ou plusieurs des options suivantes sont respectées :
a) le système comprend en outre un capteur de localisation (118, 516) configuré pour surveiller une localisation géographique ;
dans lequel l'événement déclencheur prédéterminé comporte la localisation géographique captée par le capteur de localisation (118, 516) étant une localisation géographique inacceptable ;
b) le système comprend en outre un capteur de température (118, 516) configuré pour surveiller la température ;
dans lequel l'événement déclencheur prédéterminé comporte la température captée par le capteur de température (118, 516) étant une température inacceptable ;
c) le système comprend en outre un capteur d'humidité (118, 516) configuré pour surveiller l'humidité ;
dans lequel l'événement déclencheur prédéterminé comporte l'humidité captée par le capteur d'humidité (118, 516) étant une humidité inacceptable ;
d) le système comprend en outre un capteur de pH (118, 516) configuré pour surveiller le pH ;
dans lequel l'événement déclencheur prédéterminé comporte le pH capté par le capteur de pH étant un pH inacceptable ;
e) le système comprend en outre un capteur d'ultraviolets (UV) configuré pour surveiller une exposition aux UV ;
dans lequel l'événement déclencheur prédéterminé comporte l'exposition aux UV captée par le capteur UV étant une exposition aux UV inacceptable.

13. Système selon la revendication 11 ou 12, dans lequel l'événement déclencheur prédéterminé comporte l'identification d'un numéro d'enregistrement Drug Enforcement Administration (DEA) acceptable.

14. Système selon l'une quelconque des revendications 11 à 13, comprenant en outre une étiquette de stockage de données dans celle-ci ;
dans lequel l'événement déclencheur prédéterminé comporte l'identification de données inacceptables reçues de l'étiquette.

15. Système selon l'une quelconque des revendications précédentes, dans lequel le médicament est l'un parmi kétamine, eskétamine, naloxone et sumatriptan.
